(19) Europäisches Patentamt
European Patent Office
Office européen des brevets

(11) EP 3 987 059 B1

(12) **EUROPEAN PATENT SPECIFICATION**

(45) Date of publication and mention of the grant of the patent:
**06.07.2022 Bulletin 2022/27**

(21) Application number: **21715927.6**

(22) Date of filing: **06.04.2021**

(51) International Patent Classification (IPC):
***C12Q 1/6844*** *(2018.01)*

(52) Cooperative Patent Classification (CPC):
(C-Sets available)
**C12Q 1/6844** (Cont.)

(86) International application number:
**PCT/EP2021/058946**

(87) International publication number:
**WO 2021/198533 (07.10.2021 Gazette 2021/40)**

# (54) A METHOD OF MEASURING THE PH OF A SAMPLE

VERFAHREN ZUR MESSUNG DES PH-WERTS EINER PROBE

PROCÉDÉ DE MESURE DU PH D'UN ÉCHANTILLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.04.2020 EP 20168019**
**22.09.2020 EP 20197572**

(43) Date of publication of application:
**27.04.2022 Bulletin 2022/17**

(73) Proprietor: **Diagonal Bio AB**
**223 81 Lund (SE)**

(72) Inventors:
- **PUNYANI, Kushagr**
**223 81 Lund (SE)**
- **NYBERG, Per, Andreas**
**223 81 Lund (SE)**
- **SØPSTAD, Sindre**
**3183 Horten (NO)**
- **PEACOCK, Martin**
**Royston SG8 9JL (GB)**
- **XIONG, Linhongjia**
**Royston SG8 9JL (GB)**
- **SHIN, Jae, Yen**
**223 81 Lund (SE)**

(74) Representative: **Høiberg P/S**
**Adelgade 12**
**1304 Copenhagen K (DK)**

(56) References cited:
**GB-A- 2 501 769** **JP-A- 2004 141 158**
**US-A1- 2016 130 633**

- **XIE SHUNBI ET AL: "Using the ubiquitous pH meter combined with a loop mediated isothermal amplification method for facile and sensitive detection of Nosema bombycis genomic DNA PTP1", CHEMICAL COMMUNICATIONS, vol. 50, no. 100, 1 January 2014 (2014-01-01), pages 15932-15935, XP055778172, ISSN: 1359-7345, DOI: 10.1039/C4CC06449F**
- **KWON DONGHOON ET AL: "Facile and Sensitive Method for Detecting Cardiac Markers using Ubiquitous pH Meters", ANALYTICAL CHEMISTRY, vol. 85, no. 24, 22 November 2013 (2013-11-22), pages 12134-12137, XP055778183, ISSN: 0003-2700, DOI: 10.1021/ac403329w**
- **ERIC SALM ET AL: "Electrical Detection of Nucleic Acid Amplification Using an On-Chip Quasi-Reference Electrode and a PVC REFET", ANALYTICAL CHEMISTRY, vol. 86, no. 14, 15 July 2014 (2014-07-15) , pages 6968-6975, XP055329876, ISSN: 0003-2700, DOI: 10.1021/ac500897t**
- **LEE KANG-HO ET AL: "A Sensitive Potentiometric Sensor for Isothermal Amplification-Coupled Detection of Nucleic Acids", SENSORS, vol. 18, no. 7, 14 July 2018 (2018-07-14), page 2277, XP055778216, DOI: 10.3390/s18072277**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 987 059 B1

- **CAPELETTI LARISSA BRENTANO ET AL: "Encapsulated alizarin red species: The role of the sol-gel route on the interaction with silica ma", POWDER TECHNOLOGY, ELSEVIER, BASEL (CH), vol. 237, 24 January 2013 (2013-01-24), pages 117-124, XP028991206, ISSN: 0032-5910, DOI: 10.1016/J.POWTEC.2013.01.034**
- **AOUN R ET AL: "Synthesis of a fluoresceine-derivatized fluorene and its electrogenerated copolymers with fluorene: New pH indicators", SYNTHETIC METALS, ELSEVIER SEQUOIA LAUSANNE, CH, vol. 158, no. 21-24, 1 December 2008 (2008-12-01), pages 790-795, XP025804211, ISSN: 0379-6779, DOI: 10.1016/J.SYNTHMET.2008.05.002 [retrieved on 2008-06-24]**
- **BARTOSIK MARTIN ET AL: "Genomagnetic LAMP-based electrochemical test for determination of high-risk HPV16 and HPV18 in clinical samples", ANALYTICA CHIMICA ACTA, ELSEVIER, AMSTERDAM, NL, vol. 1042, 11 August 2018 (2018-08-11), pages 37-43, XP085513458, ISSN: 0003-2670, DOI: 10.1016/J.ACA.2018.08.020**


Remarks:

The file contains technical information submitted after the application was filed and not included in this specification

(52) Cooperative Patent Classification (CPC): (Cont.)

C-Sets

**C12Q 1/6844, C12Q 2523/115, C12Q 2527/119, C12Q 2527/137, C12Q 2565/607**

SEQUENCE LISTING

<110> Diagonal Pharma AB

<120> A Method of Measuring the pH of a Sample

<130> P5625PC00

<150> EP20197572.9
<151> 2020-09-22

<150> EP20168019.6
<151> 2020-04-03

<160> 8

<170> BiSSAP 1.3.5

<210> 1
<211> 269
<212> DNA
<213> SARS coronavirus

<223> "ORF1 fragment of SARS-CoV-2"

<220>
<223> ORF1 fragment of SARS-CoV-2

<400> 1

**ccctatgtgt tcatcaaacg ttcggatgct cgaactgcac ctcatggtca tgttatggtt 60**

**gagctggtag cagaactcga aggcattcag tacggtcgta gtggtgagac acttggtgtc 120**

**cttgtccctc atgtgggcga aataccagtg gcttaccgca aggttcttct tcgtaagaac 180**

**ggtaataaag gagctggtgg ccatagttac ggcgccgatc taaagtcatt tgacttaggc 240**

**gacgagcttg gcactgatcc ttatgaaga 269**

<210> 2
<211> 237
<212> DNA
<213> SARS coronavirus

<223> "Gene N fragment of SARS-CoV-2"

<220>
<223> Gene N fragment of SARS-CoV-2

<400> 2

**atgaccaaat tggctactac cgaagagcta ccagacgaat tcgtggtggt gacggtaaaa 60**

**tgaaagatct cagtccaaga tggtatttct actacctagg aactgggcca gaagctggac 120**

**ttccctatgg tgctaacaaa gacggcatca tatgggttgc aactgaggga gccttgaata 180**

**caccaaaaga tcacattggc acccgcaatc ctgctaacaa tgctgcaatc gtgctac 237**

<210> 3
<211> 48
<212> DNA
<213> Artificial Sequence

<223> "Forward Inner Primer (FIP)"

<220>
<223> Forward Inner Primer (FIP)

<400> 3
aggtgagggt tttctacatc actatattgg aacaagcaaa ttctatgg 48

<210> 4
<211> 43
<212> DNA
<213> Artificial Sequence

<223> "Backward Inner Primer (BIP)"

<220>
<223> Backward Inner Primer (BIP)

<400> 4
atgggttggg attatcctaa atgtgtgcga gcaagaacaa gtg 43

<210> 5
<211> 20
<212> DNA
<213> Artificial Sequence

<223> "Forward Outer Primer (F3"

<220>
<223> Forward Outer Primer (F3

<400> 5
ccactagagg agctactgta 20

<210> 6
<211> 18
<212> DNA
<213> Artificial Sequence

<223> "Backward Outer Primer (B3)"

<220>
<223> Backward Outer Primer (B3)

<400> 6
tgacaagcta caacacgt 18

<210> 7
<211> 25
<212> DNA
<213> Artificial Sequence

<223> "Forward Loop Primer (FL)"

<220>
<223> Forward Loop Primer (FL)

<400> 7

cagtttttaa catgttgtgc caacc 25

<210> 8
<211> 21
<212> DNA
<213> Artificial Sequence

<223> "Backward Loop Primer (BL)"

<220>
<223> Backward Loop Primer (BL)

<400> 8
tagagccatg cctaacatgc t 21

## Description

The present invention provides a more sensitive and accurate method of monitoring the pH of a solution, wherein the pH of the solution is quantified as a function of the electrochemical response of the solution in a two or three-electrode electrochemical cell, wherein the solution comprises a compound capable of undergoing a change in its oxidation state and/or structural conformation as a function of the pH of the solution.

The present invention also provides highly accelerated methods and processes enabling analysis of specific polynucleotide sequences in a sample, e.g. a biological sample. The methods of the inventions are, for example, useful for rapid screening of a large amount of samples in a point-of-care setting.

## Background

Nucleic acid amplification methods are crucial for medical and environmental diagnostics and often considered to be the gold standards in several diagnostic applications. Traditional methods such as Polymerase Chain Reaction, Quantitative Polymerase Chain Reaction, Reverse Transcriptase Polymerase Chain Reaction and Quantitative Reverse Transcriptase Polymerase Chain Reaction are largely dependent on thermal cycling for actuation of amplification due to the need of different temperatures for denaturation, annealing and extension by commonly used polymerases. The technological issues and the resultant high implementation cost of these methods has hampered their point-of-care use. Isothermal amplification methods such as Branched Rolling Circle Amplification and Loop Mediated Isothermal Amplification are alternatives.

Quantification of the nucleic acid amplicons and real time monitoring of amplification is indirect and requires labels, which are non-essential for the amplification reaction. These may include fluorescent dyes or chromophores attached to oligonucleotides or primers, DNA-intercalating dyes, groove binders, dyes binding to nucleotides, phosphate groups, ribose or deoxyribose groups and the nitrogen bases endogenous to the nucleic acids. These dyes or complexes may exhibit a spectral shift upon binding to the DNA, undergo fluorescence resonance energy transfer, and/or a change in magnitude of fluorescence when excited by light of suitable wavelengths, thereby facilitating optical detection. This method of quantification needs expensive and sensitive optics, thereby further limiting the use of molecular diagnostics for point-of-care applications.

Several of these DNA binding molecules, such as Hoechst 33258, Methylene Blue, nucleic acid-binding transition metal complexes such as ruthenium, osmium or platinum containing complexes are also electroactive and may facilitate indirect electrochemical detection by continuous sequestration of said molecules to the newly generated amplicons upon successful amplification, and the consequent reduced conductivity of the reaction mixture.

Alternative electrochemical detection methodologies involve immobilization of the enzymes, primers or probe oligonucleotides on the electrode surface thereby actuating quantification of the amplicons or byproducts. Most of these methods are either indirect or require expensive devices.

Recent development in Loop mediated isothermal amplification technology include colorimetric detection of RNA and DNA targets in a weakly buffered reaction mixture, mediated by a pH sensitive dye (WO 2017/209920, WO 2014/031783). The weak buffering due to low concentration of Tris is overcome by the protons released during amplification and results in an end-point detection indicated by the color change of the pH indicator. The method has been deployed for point-of-care testing, but suffers from long reaction times, low sensitivity for low nucleic acid concentrations, and the need of expensive optical devices for quantification.

Capeletti et al. 2013 describes various sensors encompassing silica encapsulated alizarin red species.

Aoun et al. 2008 discloses synthesis of fluorene derivatives which may be used as pH indicators.

GB 2501769 A discloses electrochemical sensors for pH measurements as well as a device and method for measuring pH using an electrochemical sensor which has an electrode comprising an anthraquinone immobilized to the electrode and a covering layer over the anthraquinone.

Bartosik et al. 2008 describes a genomagnetic LAMP-based electrochemical test for determination of two human papillomaviruses types in clinical samples.

## Summary

The present invention provides a more sensitive and accurate method of monitoring the pH of a solution, wherein the pH of the solution is quantified as a function of the electrochemical response of the solution in a three-electrode electrochemical cell, wherein the solution comprises a quinone, a quinone derivative, and/or a pH indicator, wherein the quinone, quinone derivative, and/or pH indicator is dissolved in the solution. This more sensitive and accurate method has been found to provide rapid and accurate results in determining whether or not a target polynucleotide is present in a sample and also quantifying the target polynucleotide present in the sample.

The present invention, therefore, also provides a method of quantifying a target polynucleotide in a sample.

The method can be performed with high sensitivity without the need of optical devices for quantification and with a low reaction time. In particular, the quantification of the target polynucleotide can be derived from the rate of change of the electrochemical response (e.g., current and/or potential and/or impedance) of the electrochemical cell, such as the three-electrode electrochemical cell.

Thus, the present invention provides highly accelerated methods and processes enabling analysis of specific polynucleotide sequences in a sample. The methods are indeed useful for rapid screening of a large amount of samples in a point-of-care setting.

This is made possible because of inventive exploration of nucleic acid amplification techniques (such as LAMP) combined with electrochemical quantification of a signaling substance using an electrochemical cell, such as a three-electrode electrochemical cell or an electrochemical cell having multiple film electrodes.

The invention is defined in the claims attached hereto.

According to the first aspect of the invention there is provided a method of measuring the pH of a solution, wherein the method comprises the steps of:

- providing a solution comprising a quinone, a quinone derivative, and/or a pH indicator, wherein the quinone, quinone derivative, and/or pH indicator is dissolved in the solution;
- applying the solution to a three-electrode electrochemical cell;
- measuring an electrochemical response of the electrochemical cell; and
- quantifying the pH of the solution as a function of the electrochemical response of the electrochemical cell, wherein the response correlates with the electrochemical state of the quinone, quinone derivative and/or pH indicator.

According to another aspect of the invention there is provided a use of a three-electrode electrochemical cell for measuring the pH of a solution, said use comprising:

- providing a solution comprising a quinone, a quinone derivative, a pH indicator, or combinations thereof, wherein the quinone, quinone derivative, and/or pH indicator is dissolved in the solution;
- applying the solution to the three-electrode electrochemical cell;
- measuring an electrochemical response of the electrochemical cell; and
- quantifying the pH of the solution as a function of the electrochemical response of the electrochemical cell, wherein the response correlates with the electrochemical state of the quinone, quinone derivative and/or pH indicator.

According to another aspect of the invention there is provided a use of a three-electrode electrochemical cell for monitoring a nucleic acid amplification reaction, said use comprising:

- providing a solution comprising a quinone, a quinone derivative, a pH indicator, or combinations thereof, wherein the quinone, quinone derivative, and/or pH indicator is dissolved in the solution;
- providing a LAMP mixture comprising: a sample comprising a target polynucleotide, at least two, such as at least four primers each flanking the target polynucleotide and LAMP reagents;
- applying the solution and the LAMP mixture to the three-electrode electrochemical cell;
- measuring an electrochemical response of the electrochemical cell; and
- quantifying the pH of the solution as a function of the electrochemical response of the electrochemical cell, wherein the response correlates with the electrochemical state of the quinone, quinone derivative and/or pH indicator.

According to another aspect of the disclosure there is provided a system for measuring the pH of a solution, the system comprising:

a. a potentiostat;
b. a measuring kit, said kit comprising:

i. a first receptacle comprising a three-electrode electrochemical cell, and a second receptacle comprising a quinone, a quinone derivative, and/or a pH indicator, or
ii. a first receptacle comprising a three-electrode electrochemical cell and a quinone, a quinone derivative, and/or a pH indicator,

wherein the quinone, quinone derivative, and/or pH indicator are dissolved in an aqueous solution in the first or in the second receptacle, and wherein said aqueous solution comprises at least four primers configured to flank a target polynucleotide sequence and LAMP reagents; and
c. a heating unit, configured for heating the first receptacle, such as for heating the first receptacle to a temperature

within the range of 59°C to 75°C.

The invention is defined by the claims. Any subject matter falling outside the scope of the claims is provided for information purposes only

**Description of Drawings**

**Figure 1:** A schematic drawing of LAMP primers and the polynucleotide sequences they anneal to. Arrows indicate where a primer anneals and inverse shades of grey indicates complementarity. FIP: forward inner primer. BIP: backward inner primer. F3: forward outer primer. B3: backward outer primer. FL: forward loop primer. BL: backward loop primer.

Figure 2: (A) A fluid or fluidized or resuspended biological sample, in a suitable carrier or transport medium (if any) after suitable processing (if any) is mixed with (B) LAMP primers and (C) WarmStart Colorimetric LAMP 2X Master Mix from New England Biolabs Inc, and immediately added on to (D) a pH sensing screen printed electrode or a screen printed electrode which is at 59- 75°C and connected to a potentiostat and voltammetric or other electrochemical measurements are run for 1-90 minutes. The (E) change in signal over time is measured. A zero change in signal in 1-90 minutes compared to a parallelized or pre-calibrated non-template control indicates (F) the absence of the template nucleic acid. A significant non-zero change in signal indicates (G) the presence of the template nucleic acid, and the rate of change of this value is used to quantify the amount of the template nucleic acid in the biological sample using a pre-determined calibration curve specific to the LAMP primers and the biological sample and its processing.

**Figure 3:** A cyclic voltammogram of phenol red. Three peaks are identified at -0.35V, 0.2V and 0.55V respectively.

**Figure 4:** Cathodic cyclic voltammetry scans of solutions containing 1.0 mM phenol red buffered at pH 7.9 in 0.1 M PBS on a bare glassy carbon electrode at scan rate of 100, 300, 500, 700, and 900 mV/s, initiated from 1.5 V vs. Ag/AgCl. Inset plot shows the current dependence upon scan rate and square root of scan rate, respectively.

**Figure 5:** Cathodic cyclic voltammetry scans of solutions containing 1.0 mM phenol red (buffered at pH 7.9 in 0.1 M PBS) on a bare glassy carbon electrode over different switching potentials, at a scan rate of 100 mV/s initiated from 1.5 V vs. Ag/AgCl. Inset plot reveals the ratio of ipc/ipa near -0.75 V varied according to the switching potential.

**Figure 6:** Anodic cyclic voltammetry scans of solutions containing 1.0 mM phenol red buffered at pH (a) 3.9, (b) 7.9, and (c) 11.9 with 0.1 M PBS on a bare glassy carbon electrode, respectively; (d) represents the scan of the solution (b) initiated from more positive potential. The cathodic cyclic voltammetry scans of those corresponding pH solutions are noted as a', b', and c'.

**Figure 7a-d:** Example of squarewave voltammetry of phenol red in the pH range of 6.5 to 8.5, and at different concentrations of Phenol Red. Figures 7b and 7d show peak positions (voltage) and heights (current) versus concentrations of phenol red.

**Figure** 8: An example of in-house algorithm for correlating the pH with phenol red output signal, where an optimised algorithm is used to nest both the peak potential (peak identified at around 0.5V (vs Ag/AgCl) and peak current.

**Figure** 9: Voltammogram showing the peaks observed in cyclic voltammetry of phenol red solution.

**Figure 10:** Schematic showing the design of sensor environment controller.

**Detailed description**

**Definitions**

The term "nucleotide sense strand" as used herein refers to a DNA or RNA strand that is complementary to a nucleotide antisense strand.

The term "nucleotide antisense strand" as used herein refers to a DNA or RNA strand that is complementary

to a nucleotide sense strand.

The term "forward inner primer" or "FIP" as used herein refers to an oligonucleotide having a 3' end and a 5' end, wherein the oligonucleotide comprises a first part at the 3' end of the oligonucleotide and a second part at the 5' end of the oligonucleotide. Generally, the first part is complementary to a nucleotide sequence in the antisense strand of the target polynucleotide and the second part is complementary to a nucleotide sequence in the sense strand of the target polynucleotide. In other words, the first part may be a F2 region and the second part may be an F1c region. The F2 region is complementary to the F2c region in the target DNA sequence while the F1c region is complementary to the F1 region in the target DNA sequence (Fig. 1).

The term "backward inner primer" or "BIP" as used herein refers to an oligonucleotide having a 3' end and a 5' end, wherein the oligonucleotide comprises a first part at the 3' end of the oligonucleotide and a second part at the 5' end of the oligonucleotide. Generally, the first part is complementary to a nucleotide sequence in the sense stand of the target polynucleotide and the second part is complementary to a nucleotide sequence in the antisense strand of the target polynucleotide. In other words, the first part may be a B2 region and the second part may be a B1c region. The B2 region is complementary to the B2c region in the target DNA sequence while the B1c region is complementary to the B1 region in the target DNA sequence (Fig. 1).

The term "forward outer primer" or "F3" as used herein refers to an oligonucleotide, which is complementary to a nucleotide sequence in the antisense stand of the target polynucleotide. Thus, F3 may comprise a region that is complementary to a F3c region in the target DNA sequence (Fig. 1).

The term "backward outer primer" or "B3" as used herein refers to an oligonucleotide which is complementary to a nucleotide sequence in the sense strand of the target polynucleotide. Thus, B3 may comprise a region that is complementary to a B3c region in the target DNA sequence (Fig. 1).

The term "forward loop primer" or "FL" as used herein refers to an oligonucleotide that is complementary to a polynucleotide sequence in the sense strand of the target polynucleotide. Thus FL is complementary to a section on the target DNA sequence designated FLc (Fig. 1).

The term "backward loop primer" or "BL" as used herein refers to an oligonucleotide that is complementary to a polynucleotide sequence in the antisense strand of the target polynucleotide. Thus, BL is complementary to a section on the target DNA sequence designated BLc (Fig. 1).

The term "nucleic acid amplification" as used herein refers to any nucleic acid step/method/protocol that is used to replicate and multiply a particular nucleic acid sequence in a sample.

The term "LAMP" refers to loop-mediated isothermal amplification. LAMP is a reaction for amplification of nucleic acids. LAMP uses 4 (or 6) primers targeting 6 (or 8) regions within or surrounding the target sequence (Fig. 1). The method relies on isothermal conditions, i.e. it is carried out at a constant temperature and does not require a thermal cycler. Briefly, LAMP amplification of the target gene occurs when it is incubated with the target specific primers FIP, BIP, F3 and B3, and a polymerase at a constant temperature in the range of 59 and 75°C. Inclusion of forward and backward loop primers, FL and BL respectively, may be added to the LAMP amplification. The amplification product can be detected by any appropriate method. The outcome of the method can be correlated with the number of DNA copies produced during LAMP amplification and thus serve as the basis for quantification of the amount of DNA present in the sample. Quantitative detection can be performed both by end-point and real-time measurements. The DNA may also be cDNA:

The term "RT-LAMP" refers to reverse transcription loop-mediated isothermal amplification. RT-LAMP combines LAMP with a reverse transcription step to allow the detection of RNA.

The term "nucleic acid amplification reagents" as used herein refers to agents which are added to a sample in order to effect amplification of any target poynucleotide(s) within the sample.

The term "LAMP reagents" as used herein refers to agents, which are added to a LAMP in addition to a sample and at least four primers. The LAMP reagents comprise at least nucleotides and a nucleic acid polymerase. In addition, the LAMP reagents may comprise other compounds such as salt(s) and buffer(s). The nucleic acid polymerase preferably has a high strand displacement activity and replication activity.

The term "signaling substance" as used herein refers to any physical quantity of the amplification reagents (such as LAMP reagents) or sample which undergoes change, or an agent produced, consumed or undergoing a phase change during nucleic acid amplification and the amount of which can be detected and thus used to demonstrate or quantify the amplification reaction, such as a production of $H^+$-ions, or a drop in pH or conductivity of the sample/reaction mixture.

The term "target sequence" as used herein refers to any nucleic acid sequence, which is desired to detect. In an embodiment, the target sequence is a nucleic acid sequence, preferably a nucleic acid sequences which can be amplified by any nucleic acid amplification technology, such as LAMP, preferably wherein the target nucleic acid sequence is amplified using at least four primers flanking the target sequence.

The term "target polynucleotide" as used herein encompasses the term "target nucleic acid" and the two definitions may be used interchangeably unless expressly stated otherwise.

The term "electrochemical device" as used herein refers to any device capable of study an analyte by measuring the potential (volts) and/or current (amperes) in an electrochemical cell containing the analyte. Typically, the three main categories for measuring using an electrochemical device are potentiometry (the difference in electrode potentials is measured), coulometry (the cell's current is measured over time), and voltammetry (the cell's current is measured while actively altering the cell's potential). The term "electrochemical device" as used herein may also be used interchangeably with the term "three-electrode electrochemical cell" when referring to the first aspect of the invention. For the avoidance of doubt, unless expressly stated to the contrary, all embodiments as described herein with reference to the "electrochemical device" refer to both the electrochemical device of the second aspect of the invention and the three-electrode electrochemical cell of the first aspect of the invention.

The term "three-electrode electrochemical cell" as used herein refers to an electrochemical cell comprising three different electrodes, being a working electrode, a counter electrode and a reference electrode. In use, all three electrodes of the electrochemical cell are in contact with the solution being analysed. During three-electrode experiments, charge flow (current) primarily occurs between the working electrode and the counter electrode while the potential of the working electrode is measured with respect to the reference electrode.

The term "two-electrode electrochemical cell" as used herein refers to an electrochemical cell comprising two electrodes, wherein one electrode is a working electrode and the second electrode is a combined reference and counter electrode. That is to say, the second electrode is a variation of a three-electrode cell wherein the reference and counter electrode are shorted and act as one electrode, i.e. they are the same electrode.

The term "electrode" as used herein refers to an electrically conductive material (often made from carbon, metal, or a composite) that provides a path for current to flow into, or out of, an electrochemical system. During an electrochemical measurement, some portion of the electrode surface is in direct contact with an ionically-conductive medium, or electrolyte, through which charge is transferred between other electrodes.

By the term "quinone" as used herein, this refers to organic compounds that are cyclic organic compounds containing two carbonyl (C=O) groups either adjacent or separated by a vinylene (-CH=CH-) group.

By the term "quinone derivative" as used herein, this refers to compounds that are derived from quinones as defined above wherein one or more of the hydrogen atoms in the cyclic backbone of the compound has been replaced by other atoms or radicals.

As used herein, the term "squarewave voltammetry" refers to a form of linear potential sweep voltammetry that uses a combined square wave and staircase potential applied to a stationary electrode.

As used herein, the term "electrochemical response" refers to any measurable process that either causes or is accompanied by the passage of an electric current. Such measurable electrochemical responses include measuring the potential, the current, or the impedance of a system, or indeed a combination of any of these measurements.

The term "pH indicator" as used herein refers to a halochromic chemical compound, which is a chemical compound that changes colour when pH changes occur, and which, if added in small amounts to a solution, allow the pH (acidity or basicity) of the solution to be determined visually.

The term catechol, as used herein, comprises all groups having a structural motive according to the following general formula I:

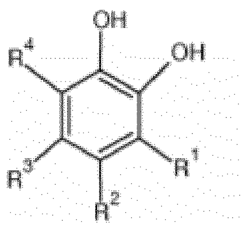

Formula I

Thereby, residues $R^1$, $R^2$, $R^3$ and $R^4$ can be absent, hydrogen or any organic or organometallic residue. Preferred residues are aliphatic or aromatic chains (such as, e.g., $C_1$-$C_{10}$ aliphatic chains or $C_6$-$C_{20}$ aromatic residues) that can optionally be interrupted or substituted by moieties containing nitrogen, oxygen and/or sulfur atoms, e.g., by $-NH_2$, -NH-, -OH, =O, -O-, -SH and/or -S-S-. Residues $R^1$, $R^2$, $R^3$ and $R^4$ can have independently from each other the same meaning or different meanings in each case. The term catechol, as used herein, also refers to a substituted orthodihydroxybenezene derivative. Two different isomeric conformations are represented by Catechol is also known as pyrocatechol and benzene-1,2-diol.

The term "catecholic group" also refers to the oxidized form of catechol and its derivatives that is also known as quinone and corresponds to the following general formula II:

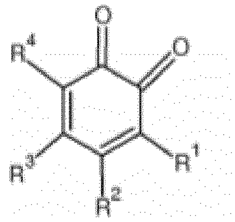

Formula II

The residues $R^1$, $R^2$, $R^3$ and $R^4$ can have the same meaning as previously explained.

The term "receptacle" as used herein relates to a vessel such as a container suitable for accommodating a liquid, such as an aqueous solution. A receptacle usually comprises an aperture and a lid that can be used to close that aperture. A receptacle according to the present disclosure may also comprise three-electrode electrochemical cell.

**Method of Measuring the pH of a Solution**

According to one aspect of the invention there is provided a method of measuring the pH of a solution, wherein the method comprises the steps of:

- providing a solution comprising a compound capable of undergoing a change in its oxidation state and/or structural conformation as a function of the pH of the solution, said compound being a quinone, a quinone derivative, and/or a pH indicator, wherein the quinone, quinone derivative, and/or pH indicator is dissolved in the solution;
- applying the solution to a three-electrode electrochemical cell;
- measuring an electrochemical response of the electrochemical cell; and
- quantifying the pH of the solution as a function of the electrochemical response of the electrochemical cell, wherein the response correlates with the electrochemical state of the quinone, quinone derivative and/or pH indicator.

According to another aspect of the invention there is provided a method of measuring the pH of a solution, wherein the method comprises the steps of:

- providing a solution comprising a quinone, a quinone derivative, and/or a pH indicator, wherein the quinone, quinone derivative, and/or pH indicator is dissolved in the solution;
- applying the solution to a three-electrode electrochemical cell;
- measuring an electrochemical response of the electrochemical cell; and
- quantifying the pH of the solution as a function of the electrochemical response of the electrochemical cell, wherein the response correlates with the electrochemical state of the quinone, quinone derivative and/or pH indicator.

In an embodiment, the solution comprises a plurality of compounds capable of undergoing a change in their oxidation state and/or structural conformation as a function of the pH of the solution. That is to say that the solution may comprise a plurality of structurally different compounds capable of undergoing a change in their oxidation state and/or structural conformation as a function of the pH of the solution, such as two or more compounds, for example three, four, five, six, seven, eight, nine or ten or more compounds.

In another embodiment the compound capable of undergoing a change in its oxidation state and/or structural conformation as a function of the pH of the solution is a catechol, a catechol derivative and/or a compound comprising a catecholic group.

In another embodiment the compound capable of undergoing a change in its oxidation state and/or structural conformation as a function of the pH of the solution is a quinone, a quinone derivative, a pH indicator, or combinations thereof.

In a further embodiment the compound capable of undergoing a change in its oxidation state and/or structural conformation as a function of the pH of the solution is a quinone or a quinone derivative selected from the list consisting of 1,2-benzoquinone, 1,4-benzoquinone, 1,4-naphthoquinone, 9,10-anthraquinone, and derivatives and combinations thereof.

In another embodiment the compound capable of undergoing a change in its oxidation state and/or structural conformation as a function of the pH of the solution is a pH indicator selected from the list consisting of malachite green oxalate, brilliant green, eosin yellowish, erythrosine B, methyl green, methyl violet, picric acid, cresol red, crystal violet, m-Cresol purple, thymol blue, p-Xylenol blue, Eosin (bluish), quinaldine red, 2,4-dinitro phenol, 4-(dimethylamino) azobenzol, bromochlorophenol blue, bromophenol blue, congo red, methyl orange, bromocresol green, 2,5-dinitrophenol, alizarin sulphonic acid, methyl red, chlorophenol red, litmus, bromocresol purple, bromophenol red, 4-nitrophenol, bromoxylenol blue, bromothymol blue, phenol red, 3-nitrophenol, neutral red, cresol red, 1-naphtholphthalein, m-cresol purple, thymol blue, p-xylenol blue, phenolphthalein, thymolphthalein, alkali blue, alizarin yellow GG, indigo carmine, epsilon blue, titan yellow, and combinations thereof. In an embodiment, the solution comprises at least two of the compounds listed in this paragraph, for example at least three, four or five compounds listed in this paragraph.

In one embodiment of the present disclosure, two or more compounds, said compounds being a catechol, a catechol derivative, a compound comprising a catechol group, a quinone, a quinone derivative or a pH indicator, are provided in the same aqueous solution. Thus, pH measurements and quantification can be conducted on a larger pH scale, such as between pH 5 and 8, such as between pH 4 and 8, such as between pH 3 and 8, such as between pH 3 and 9, such as between pH 4 and 9, such as between pH 5 and 9, such as between pH 6 and 9, such as across all pH range.

In one embodiment of the present disclosure, the three-electrode electrochemical cell does not comprise a quinhydrone electrode. In fact, a quinhydrone electrode cannot effectively measure pH of solutions where the test solution itself causes a change in open circuit potential (OCP) over time due to biochemical or chemical reactions. Further, quinhydrone electrodes suffer from a salt error. The functioning of quinhydrone electrode may be impaired by the presence of oxidizing and reducing agents and the quinhydrone electrode is poisoned by traces of metals such as copper, silver, and other metals below antimony in the electromotive series, which may also interferer with pH measurements if complexing agents are present in the solution to be analyzed.

More importantly, a quinhydrone electrode is not suitable for usage in a three-electrode electrochemical cell, because quinhydrone and its aqueous dissociation products cannot be electrochemically analyzed, such as distinguished, in a three-electrode electrochemical cell. Thus, a quinhydrone electrode is not suitable for use in the methods and systems disclosed herein.

Hence, in one embodiment of the present disclosure the quinone, quinone derivative, and/or pH indicator is not quinhydrone.

Using a catechol, a catechol derivative and/or a compound comprising a catecholic group, such as a quinone or quinone derivative, such as any one of the listed specific compounds, for example phenol red, provides several advantages with quantification of the pH of a solution. One of the advantages is a high resolution in the pH measurement, in particular a higher resolution compared to quantifying pH with a standard pH meter that measures hydrogen-ion activity in aqueous solutions. This is due to the rich redox chemistry that characterizes a catechol, a catechol derivative and/or a compound comprising a catecholic group, such as a quinone or quinone derivative, such as any one of the listed specific compounds, for example phenol red, as provided in detail in Example 3 of the present disclosure.

Thus, the method and system disclosed in the present disclosure provide accurate and instantaneous pH measurements as the response time of the pH measurement is not dependent on the diffusion of ions, for example protons, across a semi-permeable membrane or an adsorbing membrane, but instead based on measuring an electrochemical response that correlates with the electrochemical state of a catechol, a catechol derivative and/or a compound comprising a catecholic group, such as a quinone or quinone derivative, or a pH indicator.

In a further embodiment the compound capable of undergoing a change in its oxidation state and/or structural conformation as a function of the pH of the solution is phenol red.

In another embodiment the compound capable of undergoing a change in its oxidation state and/or structural conformation as a function of the pH of the solution is a weak acid, for example a weak acid having a pKa of from about 2 to about 14, such as a pKa from about 4 to about 12, for example a pKa of from about 6 to about 10.

According to the present disclosure, the compound capable of undergoing a change in its oxidation state and/or structural conformation as a function of the pH of the solution is dissolved in the solution. That is to say, the compound capable of undergoing a change in its oxidation state and/or structural conformation as a function of the pH of the sample solution is not bound, in particular not irreversibly bound, either chemically or through any physical means, to any of the electrodes of the two or three-electrode chemical cell.

In another embodiment the solution is an aqueous solution. In particular, the solution is an aqueous solution comprising at least 90 vol.% water, such as at least 91, 92, 93, 94, 95, 96, 97, 98 or 99 vol.% water. For example, the solution is an aqueous solution comprising greater than 99 vol.% water.

In an embodiment the potential of the three-electrode electrochemical cell is measured via potentiodynamic electrochemistry, such as cyclic squarewave voltammetry, squarewave voltammetry, linear sweep voltammetry, cyclic voltammetry or open circuit potentiommetry.

In an embodiment, the electrochemical cell is a three-electrode chemical cell and the potential of the cell is measured by cyclic squarewave voltammetry, squarewave voltammetry, linear sweep voltammetry, or cyclic voltammetry.

In another embodiment, the squarewave voltammetry step is run at a scan range of about -2 to about 2 V vs. Ag/AgCI, such as from about -1 to about 1 V vs. Ag/AgCI, for example from about -0.6 to about 0.6 V vs. Ag/AgCI.

In a further embodiment, the squarewave voltammetry step is run at a frequency of about 1 to about 50 Hz, such as from about 5 to about 20 Hz.

In an embodiment, the squarewave voltammetry step is run at a potential step of from about 1 to about 20mV,

such as from about 1 to about 10mV.

In another embodiment, the squarewave voltammetry step is run at an amplitude of from about 1 to about 50 mV, such as from about 1 to about 25 mV, for example from about 5 to about 20 mV.

In a further embodiment the concentration of the compound capable of undergoing a change in its oxidation state and/or structural conformation as a function of the pH of the solution within the solution is from about 1 μM to about 1000 μM, such as from about 1 μM to about 500 μM, for example from about 5 μM to about 250 μM, in particular from about 50 μM to about 200 μM, for example from about 50 μM to about 150 μM, such as from about 50 μM to about 100 μM.

In an embodiment the step of quantifying the pH of the solution as a function of the electrochemical response of the electrochemical cell is performed *via* application of a regression algorithm, such as a linear regression algorithm. Without wishing to be bound by theory, the linear regression algorithm correlates the pH with the function of redox potential and current in a linear fashion.

In another embodiment the solution comprises a buffer.

In an embodiment, the buffer comprises trisaminomethane in an amount of from 20 to 450 μM.

In a further embodiment, the buffer comprises $(NH_4)_2SO_4$ in an amount of from 5 to 20 mM.

In another embodiment, the buffer comprises KCl in an amount of from 25 to 100 mM.

In an embodiment, the buffer comprises $MgSO_4$ in an amount of from 5 to 20 mM.

In an embodiment, the buffer comprises deoxynucleoside triphosphate in an amount of from 1 to 5 mM.

In another embodiment, the buffer comprises tween-20 in an amount of from 0.05 to 1 %v/v.

Another aspect of the present disclosure relates to a use of a quinone, a quinone derivative, and/or a pH indicator, for quantifying the pH of a solution, wherein the quinone, quinone derivative, and/or pH indicator is dissolved in the solution,

wherein the solution is in an electrochemical cell, and
wherein the pH is quantified by measuring an electrochemical response in the electrochemical cell, wherein the response correlates with the electrochemical state of the quinone, quinone derivative and/or pH indicator.

**Methods of quantifying a target polynucleotide**

As defined further below, the method of the first aspect of the invention may also comprise a step of providing a sample. In an embodiment, the sample is provided in the solution, that is to say the solution may comprise the sample. The sample may be any sample. In a preferred embodiment, the sample is a biological sample. None limiting examples hereof are body fluid samples or tissue samples as defined further below.

It is envisaged that the sample may or may not comprise a target polynucleotide/target nucleic acid. Therefore, in an embodiment of the first aspect of the invention, the method allows for determining the presence of a target polynucleotide in the solution. That is to say, the method allows for measuring the pH of the solution and from this allows for determining whether or not a target polynucleotide is present in the solution.

In an embodiment the solution further comprises primers and nucleic acid amplification reagents, preferably wherein the nucleic acid amplification reagents are LAMP reagents.

In a further embodiment the nucleic acid amplification reagents comprise a signaling substance, or are capable of releasing a signaling substance, or comprise both a signaling substance and a substance capable of release a signaling substance.

In another embodiment the signaling substance is $H^+$ and/or $H_3O^+$.

In another embodiment the LAMP reagents comprise a reverse transcriptase.

In a further embodiment the primers comprise a forward inner primer, a backward inner primer, a forward outer primer and a backward outer primer.

In an embodiment the LAMP amplification is performed with an additional pair of loop primers, such as forward loop primer and/or backward loop primer.

In an embodiment the FIP/BIP is present in the solution in an amount of 1.6 μM, the F3/B3 is present in an amount of 0.2 μM, and the LoopF/B is present in an amount of 0.4 μM.

In an embodiment *Bst* DNA polymerase is present in the solution in an amount of 0.32 U/μL and reverse transcriptase is present in an amount of 0.3 U/μL.

In an embodiment the method comprises a step of performing a nucleic acid amplification step (e.g. an isothermal nucleic acid amplification step), preferably a plurality of nucleic acid amplification steps. The nucleic acid amplification step(s) is/are advantageously performed after the solution is applied to the two or three-electrode electrochemical cell. In a further embodiment, the steps of performing the nucleic acid amplifications, measuring the electrochemical response of the electrochemical cell and quantifying the pH of the solution as a function of the electrochemical response of the electrochemical cell may be performed sequentially in this order, or in any other order, or may be performed simultaneously, and the steps may also be performed simultaneously and continuously.

It is envisaged that any nucleic acid amplification step/protocol/method may be used in the method of the first aspect of the invention and such amplification methods may be selected from the list consisting of polymerase chain reaction (PCR) (including nested (n), quantitative (q) or real-time reverse transcriptase (RT) PCR), LAMP as defined elsewhere herein, Rolling Circle Amplification, and quantitative nucleic acid sequencebased amplification (QT-NASBA).

In a particular embodiment the nucleic acid amplification step/protocol/method is isothermal, such as wherein the nucleic acid amplification is isothermal and carried out at a temperature in the range of from 40 to 80°C, such as from 50 to 70°C, for example from 57 to 65°C.

In an embodiment, the nucleic acid isothermal amplification step is a LAMP step or a Rolling Circle Amplification step.

In a further embodiment the nucleic acid amplification step(s) is/are a LAMP step. For the avoidance of doubt, where only one nucleic acid amplification step is performed then in this embodiment only one LAMP step is performed. However, when multiple nucleic acid amplification steps are envisaged then multiple LAMP steps are performed.

In an embodiment the method is for quantifying the amount of a target polynucleotide in the solution or sample provided in the solution.

In another embodiment the step of measuring the electrochemical response of the electrochemical cell comprises measuring a change in the current and/or impedance and/or potential of the electrochemical cell due to a concentration change of the signaling substance.

In a further embodiment the method comprises a step of deriving an amplification rate of the nucleic acid from the rate of the change of the electrochemical response (e.g., current and/or impedance and/or potential) of the three-electrode electrochemical cell.

In an embodiment the step of deriving the amplification rate of the nucleic acid is performed simultaneously to the step of measuring the electrochemical response of the two or three-electrode electrochemical cell.

In a further embodiment the nucleic acid amplification step produces or consumes a signaling substance, such as $H^+$, as the nucleic acid is amplified. This is on the proviso that a nucleic acid/polynucleotide is present in the solution in the first instance. The production or consumption of the signaling substance in turn causes a change in the current and/or potential and/or impedance of the electrochemical cell due to a concentration change in the signaling substance. As a result of the change in the current and/or potential and/or impedance of the electrochemical cell, the pH value quantified also changes and from this change in pH an amplification rate of the nucleic acid/target polynucleotide may be derived. For the avoidance of doubt, the step of deriving an amplification rate is not necessary in order to determine whether or not a target polynucleotide is present in the solution as any detectable change in the current and/or potential and/or impedance of the electrochemical cell and subsequently quantified pH change would indicate the presence of the polynucleotide.

Therefore, a particular first aspect of the invention provides a method of measuring the pH of a solution and of determining the presence of a target polynucleotide in the solution, wherein the method comprises the steps of:

a. providing a solution comprising a compound capable of undergoing a change in its oxidation state or structural conformation as a function of the pH of the solution, wherein said compound is a quinone, a quinone derivative, a pH indicator, or combinations thereof, and wherein the quinone, quinone derivative, and/or pH indicator is dissolved in the solution; and a sample;
b. applying the solution to a three-electrode electrochemical cell;
c. performing at least one, or a plurality of, nucleic acid amplification steps wherein the nucleic acid amplification(s) produce or consume a signaling substance, if a target polynucleotide is present;
d. measuring the electrochemical response of the electrochemical cell, such as the change in the current, and/or potential, and/or impedance of the electrochemical cell, due to a concentration change of the signaling substance;
e. quantifying the pH of the solution and/or the change in the pH of the solution as a function of the electrochemical response of the electrochemical cell, wherein the response correlates with the electrochemical state of the quinone, quinone derivative and/or pH indicator; and
f. optionally deriving the amplification rate of a target polynucleotide in the sample, if originally present, by the rate of the change of the electrochemical response of the electrochemical cell and thereby quantifying the amount of target polynucleotide in the sample.

Therefore, a particular first aspect of the invention provides a method of measuring the pH of a solution and of determining the presence of a target polynucleotide in the solution, wherein the method comprises the steps of:

a. providing a quinone, a quinone derivative, and/or a pH indicator, wherein the quinone, quinone derivative, and/or pH indicator is dissolved in the solution;
b. applying the solution to a three-electrode electrochemical cell;
c. performing at least one, or a plurality of, nucleic acid amplification steps wherein the nucleic acid amplification(s)

produce or consume a signaling substance, if a target polynucleotide is present;
d. measuring the electrochemical response of the electrochemical cell, such as the change in the current, and/or potential, and/or impedance of the electrochemical cell, due to a concentration change of the signaling substance;
e. quantifying the pH of the solution and/or the change in the pH of the solution as a function of the electrochemical response of the electrochemical cell, wherein the response correlates with the electrochemical state of the quinone, quinone derivative and/or pH indicator; and
f. optionally deriving the amplification rate of a target polynucleotide in the sample, if originally present, by the rate of the change of the electrochemical response of the electrochemical cell and thereby quantifying the amount of target polynucleotide in the sample.

Steps c., d., e. and f. in the above paragraphs may be performed individually either sequentially in the ordered indicated, or in another order, or they may be performed simultaneously, for example simultaneously and continuously.
For the avoidance of doubt, the invention is not envisaged to be limited to whether or not a target polynucleotide is originally present in the sample. That is to say, the nucleic acid amplification step may be performed on a solution containing a sample that does not comprise a target polynucleotide and in this scenario this would result in zero, or at least a non-detectable, change in the electrochemical response (e.g. the current and/or potential and/or impedance) of the electrochemical cell, leading to a negative result for the target polynucleotide.

**Target polynucleotide**

The target polynucleotide may be any polynucleotide sequence. The target polynucleotide may in particular be a viral or bacterial polynucleotide. The target polynucleotide is ideally a stretch of DNA that is unique to a virus type or bacterial species and strand, and at the same time evolutionarily conserved enough to be stable in the genome of the particular virus or bacterium.
In one embodiment, the virus is selected from the group consisting of dsDNA viruses, ssDNA viruses, dsRNA viruses, (+)ssRNA viruses RNA, (-)ssRNA viruses RNA, ssRNA-RT viruses RNA and dsDNA-RT viruses DNA.
In a preferred embodiment the virus an (+)ssRNA virus RNA.
In one embodiment, the polynucleotide may be any polynucleotide present in an influenza A virus, influenza B virus, Zika virus, SARS-CoV virus, or MERS-CoV virus.
In one embodiment, the polynucleotide may be any polynucleotide present in a corona virus.
In one embodiment, the target polynucleotide may be any polynucleotide present within the corona virus, such as SARS-CoV-2 RNA.
In one embodiment the target polynucleotide sequence is a sequence of SEQ ID NO:1 or SEQ ID NO:2.
In one embodiment the target polynucleotide sequence comprises 70.0 percent, 71.0 percent, 72.0 percent, 73.0 percent, 74.0 percent, 75.0 percent, 76.0 percent, 77.0 percent, 78.0 percent, 79.0 percent, 80.0 percent, 81.0 percent, 82.0 percent, 83.0 percent, 84.0 percent, 85.0 percent, 86.0 percent, 87.0 percent, 88.0 percent, 89.0 percent, 90.0 percent, 91.0 percent, 92.0 percent, 93.0 percent, 94.0 percent, 95.0 percent, 96.0 percent, 97.0 percent, 98.0 percent, 99.0 percent, and 100.0 percent homology or sequence identity to SEQ ID NO:1 or SEQ ID NO:2.
The percent sequence identity (or homology) between two polypeptides may be determined using suitable computer programs, for example the GAP program of the University of Wisconsin Genetic Computing Group and it will be appreciated that percent identity is calculated in relation to polypeptides whose sequence has been aligned optimally. The alignment may alternatively be carried out using the Clustal W program (Thompson et al., (1994) Nucleic Acids Res 22, 4673-80). The parameters used may be as follows: Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05. Scoring matrix: BLOSUM.

**Sample**

In the second aspect of the invention, the method comprises a step of providing a sample. The sample may be any sample. In a preferred embodiment, the sample is a biological sample. None limiting examples hereof are body fluid samples or tissue samples.
In an even more preferred embodiment the sample comprises human, animal plant, bacterial, fungal, or protozoan cells.
In the first aspect of the invention, the method may also comprise a step of providing a sample. In an embodiment, the sample is provided in the solution, that is to say the solution may comprise the sample. The sample may be any sample. In a preferred embodiment, the sample is a biological sample. None limiting examples hereof are body fluid samples or tissue samples.
The sample may comprise a target polynucleotide as defined above. Specifically, the target polynucleotide

may be any polynucleotide sequence/nucleic acid sequence. The target polynucleotide in the sample may in particular be a viral or bacterial polynucleotide. The target polynucleotide in the sample may be a stretch of DNA that is unique to a virus type or bacterial species and strand, and at the same time evolutionarily conserved enough to be stable in the genome of the particular virus or bacterium.

In an embodiment, the sample may be an isolated DNA sample, an isolated RNA sample, a crude DNA extract sample or a crude RNA extract sample.

In one embodiment, the sample is a nasal sample, a throat sample, an anal sample, a vaginal sample, an ear draining sample, a skin surface swab sample, a urine sample, a whole blood sample, a serum sample, a plasma sample or lymph drainage sample.

In another embodiment, the sample is a tissue sample. The tissue sample may be any tissue sample, such as an epithelial tissue sample, connective tissue sample, muscle tissue sample and/or nervous tissue sample.

The methods may furthermore comprise one step of preparing the sample before applying the sample to the electrochemical device/ three-electrode electrochemical cell. The sample may be prepared by any suitable method. Examples hereof are described in Example 1 herein below.

Generally, the sample may be mixed with any suitable medium. A person skilled in the art would know a suitable medium for a specific samples. The medium may be a transport medium. The sample and transport medium can be added directly to the electrochemical device, or heated prior to applying the sample and the medium at any suitable temperature for a suitable amount of time.

In one embodiment, the sample is inoculated in transport medium. A transport medium may be any suitable medium for transporting the target polynucleotide sequence. In a preferred embodiment, the transport medium is a viral transport medium.

In another embodiment, the sample is mixed with an anticoagulant like heparin, EDTA or sodium citrate.

The sample, optionally mixed with a medium, may be lysed, for example by mechanical lysis, thermal lysis, acoustic cavitation, osmotic shock, enzymatic lysis or chemical lysis.

In one embodiment, the sample is mixed with the at least four primers and LAMP reagents prior to applying the sample to the electrochemical device.

In one embodiment, the sample is heated at 60-100 °C, for example at 65-95 °C, for example at 70-90 °C, for example at 75-85 °C.

In one embodiment, the sample is heated for at least 1 minute, such as at least 2 minutes, such as at least 5 minutes.

In one embodiment, the methods of the present invention are capable of detecting whether a target polynucleotide is present in the sample.

In another embodiment, the methods of the present invention are capable of quantifying the amount of target polynucleotide in a sample.

## Electrolytical device

In the first aspect of the invention, the method comprises the step of adding the solution to a three-electrode electrochemical cell.

In the second aspect of the invention, the method comprises a step of providing an electrochemical device comprising an electrochemical cell having multiple film electrodes.

In both aspects of the invention, the electrochemical device/ three-electrode electrochemical cell may be a planar device with film electrodes positioned in a single layer. Thereby, the film electrodes may be patterned, onto a planar substrate, with a thickness of below 2 mm, such as by screen printing or thick film technology. Screenprinted electrodes (SPE) are commonly used for forming disposable and low-cost sensors and biosensors. The electrodes of such a sensor can be manufactured by the use of a wide range of conductive inks, and in various shapes and dimensions, depending on the analytical needs. Besides their fabrication, these platforms are suitable to be customized with a variety of materials, including nanomaterials, and bio elements. Consequently, in one embodiment of the present disclosure, the electrodes are screen printed electrodes, preferably printed on a planar substrate of an inert material.

In one embodiment of the present disclosure, the electrodes of the three-electrode electrochemical cell are wire electrodes.

Preferably, the area of the electrochemical device/ three-electrode electrochemical cell is smaller than 500 $mm^2$, yet more preferably smaller than 300 $mm^2$, making the electrochemical device/ three-electrode electrochemical cell portable and suitable for a point-of-care setting. The electrodes of the electrochemical device/ three-electrode electrochemical cell may extend towards an edge of the planar substrate, and thereby form an edge connection, for electrical connection to a measurement setup, such as a potentiostat. The potentiostat may itself be a portable potentiostat, such as a hand-held potentiostat.

The electrochemical device in the second aspect of the invention may comprise multiple film electrodes, for

example a working electrode, a reference electrode and a counter electrode, wherein the electrodes preferable forms a part of a three-electrode system, such as a electrochemical three-electrode system.

A working electrode of a three-electrode system is typically the electrode at which the redox process of interest occurs. Thereby, the focus of an electroanalytical measurement is typically on a particular electrochemical reaction occurring at the working electrode.

A reference electrode typically has a stable and well-known thermodynamic potential. The high stability of the reference electrode is usually achieved by employing a redox system with constant (buffered or saturated) concentrations of the ions or molecules involved in the redox half-reaction. When used as part of a three-electrode system, current does not pass through the reference electrode.

A counter electrode, also called the auxiliary electrode, is typically used in an electrochemical system to complete the electrical circuit with the working electrode. While the redox process of interest typically occurs on the working electrode, the counter electrode may serve as a source or depository of current so as not to limit the electrochemical reactions at the working electrode.

The electrodes of the electrochemical device/ three-electrode electrochemical cell may be fabricated in a suitable material, such as gold, silver, carbon, platinum, ruthenium dioxide, or a combination thereof. The material of the electrodes may be selected individually, thereby the material of a number of the electrodes, such as each electrode, may be different. Alternatively, the electrodes may be provided in the same material. However, for providing simplicity, stability, and a capability of miniaturization, it may be a preference that the material of the reference electrode and the counter electrode is Ag/AgCl. In an alternative embodiment of the present disclosure, the material of the electrodes may be identical.

Additionally, any, or all, of the electrodes may comprise an ion-selective membrane, such an electrode may be referred to as an ion-selective electrode. Said membrane may be used to convert the activity of a specific ion dissolved in a sample into an electrical potential. The membrane may cover at least a part of the electrode, and located such that it forms an interface to a solution during a measurement. The membrane is preferably a glass membrane, typically an ion-exchange type of glass (silicate or chalcogenide). Alternatively, the membrane may be a crystalline membrane, an ion-exchange resin membrane or an enzyme membrane.

Alternatively, or additionally, to the use of an ion-selective membrane, the surface of any of the electrodes may be modified. For example, the surface of the working electrode may be chemically modified to allow for binding of hydroxide $OH^-$ and/or hydronium $H_3O^+$ ions. In an embodiment of the present disclosure, the surface of any of the electrodes may comprise a pH sensing layer of a solid-state membrane with combination of Cobalt Oxide and Iridium Oxide. Preferably, the surface of the working electrode comprises a pH sensing layer of a solid-state membrane with combination of Cobalt Oxide and Iridium Oxide.

Further modifications of the electrode surfaces include chemical modification by, ruthenium oxide, graphene platelets, exposed thiol group and/or exposed hydroxyl groups.

Preferably, the surface of any of the electrodes, for forming contact with the sample/solution, has a fixed concentration of chloride ions, such as for a true reference electrode measurement. Thereby, any, or all of the electrodes may comprise a surface having a fixed concentration of chloride ions.

In a preferred embodiment of the present disclosure, the material of the working electrode is carbon, and the material of the counter electrode and the reference electrode is Ag/AgCl.

The electrochemical device/ three-electrode electrochemical cell may be formed by screen printing of electrodes on a planar substrate. Thereby, the electrodes may be screen printed electrodes wherein the electrodes are in a single layer. The material of the planar substrate is preferably a chemically inert material, such as alumina, ceramic, glass, or an inert plastic. The electrochemical device/ three-electrode electrochemical cell preferably has a small form-factor, making it portable, and suitable for point-of-care measurements.

It is a preference that the electrochemical device/ three-electrode electrochemical cell comprises an inlet for receiving the solution to be analyzed. The inlet may be a microfluidic inlet and may further be connected to the electrochemical cell by a hydrophilic zone. The hydrophilic zone is ideally configured such that a solution provided to the microfluidic inlet is transported, by capillary action, to the electrochemical cell. Alternative means of storing and providing a solution to the electrochemical cell comprise the use of a microwell superpositioned on the electrochemical cell. The microwell may be bonded to the electrochemical device, and/or by lithography.

When the solution is provided to the electrochemical cell, it may be an advantage to apply a vapour barrier for preventing evaporation of the solution. This may especially be a requirement during longer measurement, such as at least several minutes. The vapour barrier may be formed by fluidly sealing the inlet of the electrochemical device, or the microwell, with a physical lid, a heated lid, a mineral oil, and/or a paraffin wax. Thereby, the vapour barrier act to form a fluidly sealed device comprising the solution.

In an embodiment of the present disclosure, the electrochemical device may comprise the primers and the LAMP reagents. In other words, the primers and LAMP reagents may be preloaded to the device prior to loading of the sample/solution.

In one aspect of the present disclosure, there is provided a system for measuring the pH of a solution, the

system comprising:

a. An instrument comprising a potentiostat, wherein the potentiostat is configured to measure an electrochemical response of the electrochemical cell;
b. A measuring kit, said kit comprising:

i. A first receptacle comprising a three-electrode electrochemical cell, and a second receptacle comprising a quinone, a quinone derivative, and/or a pH indicator, or
ii. A first receptacle comprising a three-electrode electrochemical cell and a quinone, a quinone derivative, and/or a pH indicator,

wherein the quinone, quinone derivative, and/or pH indicator are dissolved in an aqueous solution in the first or in the second receptacle,
wherein said aqueous solution comprises at least four primers configured to flank a target polynucleotide sequence and LAMP reagents, and

c. a heating unit, configured for heating the first receptacle, such as for heating the first receptacle to a temperature within the range of 59°C to 75°C..

The quinone, quinone derivative, and/or pH indicator of the system disclosed herein are dissolved in an aqueous solution in the first or in the second receptacle.
The quinone, quinone derivative, and/or pH indicator of the system disclosed herein are dissolved in an aqueous solution in the first receptacle, wherein said aqueous solution also comprises a nucleic acid amplification reaction system, such as at least two primers configured to flank a target sequence and LAMP reagents.
In one embodiment, the quinone, quinone derivative, and/or pH indicator of the system disclosed herein are dissolved in an aqueous solution in the second receptacle, whereas the first receptacle comprises another aqueous solution. Thus, prior to measuring the pH of the aqueous solution in the first receptacle, a suitable volume of the aqueous solution comprising the quinone, quinone derivative, and/or pH indicator is transferred to the first receptacle.
In one embodiment, the potentiostat is configured to measure an electrochemical response of the electrochemical cell.
In one embodiment, the potentiostat is configured to measure an electrochemical response, wherein the electrochemical response is representative of the oxidation state of the quinone, quinone derivative, and/or pH indicator in the first receptacle.
The system further comprises a heating unit, configured for heating the first and/or second receptacle, such as to a temperature within the in the range of from 59°C to 75°C.
The first receptacle accommodates a nucleic acid amplification reaction system, such as at least two, or at least four primers configured to flank a target polynucleotide sequence and LAMP reagents.
In one embodiment, the first receptacle comprises a microfluidic inlet for receiving a solution and/or a sample.
In one embodiment, the first and/or the second receptacles are replaced by new first and/or the second receptacles after each use.
In one embodiment, the microfluidic inlet is connected to the electrochemical cell by a hydrophilic zone, such as a porous/fibrous structure or a hydrophilic channel, configured to transport the sample by capillary action.
In one embodiment, the first receptacle and/or the second receptacle is an Eppendorf tube, a microwell, or a culture flask.
In one embodiment, the electrodes in the system of the present disclosure are as defined herein.

**LAMP amplification**

The methods of the second aspect of the invention comprise a step of performing a plurality of LAMP amplifications each comprising the sample (for example provided and/or prepared as described in the "Sample" section herein above) thereby amplifying the target polynucleotide sequence. Each LAMP amplification comprises at least four primers each set flanking the target sequence and LAMP reagents. The LAMP amplification is performed under isothermal conditions.
In an embodiment of the first aspect of the invention, the method may also comprise a step of performing a LAMP amplification, or a plurality of LAMP amplifications each comprising the sample (for example provided and/or prepared as described in the "Sample" section herein above) thereby amplifying the target polynucleotide sequence. Each LAMP amplification comprises at least four primers each set flanking the target sequence and LAMP reagents. The LAMP amplification may be performed under isothermal conditions.

The entire LAMP amplification may be prepared in a number of different manners. Any LAMP amplification known to the skilled person may be used with the invention.

One none limiting example LAMP amplification is described here: the FIP anneals to the F2c region on the antisense nucleotide strand of the target DNA. DNA synthesis is initiated by the polymerase, which displaces and releases the DNA strands. Thus, the polymerase synthesizes a strand complementary to the target DNA sequence. Next, the F3 anneals to the F3c region on this antisense strand and initiates a new round of DNA synthesis by the polymerase. The F3-mediated displacement of the antisense strand results in the formation of a stem-loop structure connected by the complementary F1c and F1 regions. Next, the BIP anneals to the DNA strand and complementary DNA is synthesized by the polymerase. Thus, the DNA transforms from a loop to a double stranded linear structure. The DNA strands separate and a dumbbell-like structure with two stem-loops, one at each end of the strand, is formed. This structure serves as the starting point for the amplification cycle. Next, the structure is converted into a stem-loop via self-primed DNA synthesis. The FIP anneals to the single stranded region in the stem-loop and initiates synthesis. In doing so, it releases the complementary strand. The released strand forms a new dumbbell-like structure due to the complementarity between the B1c-B1 and F1-F1c regions, respectively. Starting from the 3' end of the B1 region, DNA synthesis is initiated. This releases the FIP-linked complementary strand, which in turn forms yet another double-stem-loop structure due to complementarity between the F1-F1c and the B1c-B1 regions, respectively. Next, self-primed DNA synthesis starts over from the 3' end of the B1 region. The process produces amplification structures consisting of alternately inverted repeats of the target sequence.

The sample may be mixed with the primers and LAMP reagents prior to applying the sample to the electrochemical device.

In contrast to conventional PCR amplification, LAMP amplification is performed using isothermal conditions. In one embodiment, the LAMP amplification can be performed at a constant temperature at 59-75°C, such as 62-73°C, such as 64-70°C, such as 66-68°C.

## LAMP reagents

The method of both the first and second aspects of the invention encompasses performing several LAMP amplifications. The LAMP amplifications will in general comprise a sample, at least four primers flanking the target sequence and LAMP reagents. Said LAMP reagents may be any of the LAMP reagents described herein in this section.

The LAMP reagents, in general, comprise at least nucleotides and a nucleic acid polymerase. The nucleotides may be deoxy-ribonucleotide triphosphate molecules, and preferably the LAMP reagents comprise at least dATP, dCTP, dGTP and dTTP. In some cases, the LAMP reagents also comprise dUTP. The LAMP reagents also comprise a signaling substance, such as H+-ions and/or fluorescent dyes.

The nucleic acid polymerase may be any enzyme capable of catalysing templatedependent polymerisation of nucleotides, i.e. replication. The nucleic acid polymerase should tolerate the temperatures used for the LAMP amplification, and it should have catalytic activity at the elongation temperature. Several thermostable nucleic acid polymerases are known to the skilled person.

In some embodiments of both aspects of the invention, the nucleic acid polymerase has high strand displacement activity in addition to a replication activity.

The nucleic acid polymerase may be a bacterial or archaebacterial polymerase. In specific, the nucleic acid polymerase may be Escherichia coli DNA polymerase I. The nucleic acid polymerase may also be Taq DNA polymerase, which has a DNA synthesisdependent strand replacing 5'-3' exonuclease activity. Other polymerases include but are not limited to Taq, Tfi, Tzi, Tth, Pwo, Pfu, Q5®, Phusion®, One Taq®, Vent®, Deep Vent®, Klenow (exo-), Bst 2.0 and Bst 3.0 ( New England Biolabs, Ipswich, Mass.), PyroPhage® (Lucigen, Middleton, Wis.) Tin DNA polymerase, GspSSD LF DNA polymerase, Rsp (OptiGene, Horsham, UK) and phi29 polymerase.

The Taq DNA polymerase, e.g. obtained from New England Biolabs, can include Crimon LongAmp® Taq DNA polymerase, Crimson Taq DNA Polymerase, Hemo KlenTaq™, or LongAmp®Taq.

The LAMP reagents may comprise a reverse transcriptase (RT). RT is an enzyme capable of generating complementary DNA (cDNA) from an RNA template. Thus, enabling the measurement of RNA. In one embodiment, the LAMP reagents comprise a reverse transcriptase.

In addition, the LAMP reagents may comprise salts, buffers and detection means. The buffer may be any useful buffer, e.g. TRIS. The salt may be any useful salt, e.g. potassium chloride, magnesium chloride or magnesium acetate or magnesium sulfate.

The LAMP reagents may comprise a non-specific blocking agent, such as BSA, gelatin from bovine skin, beta-lactoglobulin, casein, dry milk, salmon sperm DNA or other common blocking agents.

The LAMP reagents may also comprise bio-preservatives (e.g. $NaN_3$), LAMP enhancers (e.g. betaine, trehalose, etc.) and inhibitors (e.g. RNase inhibitors). Other additives can include dimethyl sulfoxide (DMSO), glycerol, betaine (mono)-hydrate, trehalose, 7-deaza-2'-deoxyguanosine triphosphate (7-deaza-2'-dGTP), bovine serum albumin (BSA),

formamide (methanamide), tetramethylammonium chloride (TMAC), other tetraalkylammonium derivatives [e.g. tetraethylammonium chloride (TEA-Cl)]; tetrapropylammonium chloride (TPrA-Cl) or non-ionic detergent, e.g. Triton X-100, Tween 20, Nonidet P-40 (NP-40) or PREXCEL-Q.

Furthermore, the LAMP reagents may also comprise one or more additional means for detection of LAMP amplification product(s). Said means may be any detectable means, and they may be added as individual compounds or be associated with, or even covalently linked to, one of the primers. Detectable means include, but are not limited to, dyes, radioactive compounds, bioluminescent and fluorescent compounds. In a preferred embodiment, the means for detection is one or more probes.

In one embodiment, the LAMP amplification is performed using WarmStart® LAMP Kit (DNA & RNA). This kit contains a Bst 2.0 DNA polymerase, a reverse transcriptase, a nucleotide mix, a visible pH indicator, and a low-buffer solution. The kit furthermore comprises a fluorescent dye.

In another example, the LAMP amplification is performed using WarmStart® Colorimetric LAMP 2X Master Mix (DNA & RNA). This kit contains a Bst 2.0 DNA polymerase, a reverse transcriptase, a nucleotide mix and a buffer solution. The kit furthermore comprises a fluorescent dye.

**Primers flanking the target sequence**

The method of the second aspect of the invention involves the use of at least four primers that flank the target sequence.

In an embodiment of the first aspect of the invention, the method also involves the use of at least four primers that flank the target sequence.

The four primers may comprise

- a forward inner primer also referred to as FIP
- a backward inner primer also referred to as BIP
- a forward outer primer also referred to as F3
- a backward outer primer also referred to as B3

In an embodiment, the FIP has a sequence of SEQ ID NO:3.

In an embodiment, the BIP has a sequence of SEQ ID NO:4.

In an embodiment, the F3 has a sequence of SEQ ID NO:5.

In an embodiment, the B3 has a sequence of SEQ ID NO:6.

The four primers anneal to different parts of the sense and antisense stand of the target polypeptide. See the definitions of the primers in the section "Definitions" as described herein.

The primers are capable of amplifying the target polynucleotide when added to the LAMP reagents under conditions allowing amplification of said target polynucleotide.

In a preferred embodiment, the at least four primers consists of BIP, FIP, F3, and B3.

In one embodiment, the LAMP amplification is performed with an additional pair of loop primers. In one embodiment, the LAMP amplification is performed with a FL primer and a BL primer.

In an embodiment, the FL primer has a sequence of SEQ ID NO:7.

In an embodiment, the BL primer has a sequence of SEQ ID NO:8.

In one embodiment of both aspects of the invention, the LAMP amplification is performed with at least 5 primers, such as at least 6 primers, such as at least 7 primers.

In one embodiment, the LAMP amplification is performed with BIP, FIP, F3, B3, FL and BL primers.

The length of the BIP, FIP, F3, B3, FL and BL primers can depend on the sequence of the target polynucleotide. For example, the length of the primers can be adjusted to achieve a desirable activity at a specific temperature, such as in the range of 59-75°C. Thus, the length of the primers can, individually, be in the range of 10 to 100 nucleotides, for example in the range of 10 to 50 nucleotides, such as in the range of 15 to 20 nucleotides, such as in the range of 15 to 25 nucleotides, such as in the range of 15 to 30 nucleotides, such as in the range of 15 to 40 nucleotides, such as in the range of 15 to 45 nucleotides, such as in the range of 15 to 50 nucleotides in length. Tm of the primers are typically adjusted to in the range of 59 to 75°C.

Primer concentration within the aqueous phase of the LAMP amplifications can, for example, be in the range of 0.05 to 4.0 μM, such as in the range of 0.1 to 3.0 μM, such as in the range of 0.2 to 2.0 μM. One none limiting example hereof is 1.6 μM FIP, 1.6 μM BIP, 0.2 μM F3, 0.2 μM B3, 0.4 μM FL, 0.4 μM BL.

The primers in general, comprise - or even consist - of oligonucleotides. However, in some cases, the primers may comprise nucleotide analogues. Numerous nucleotide analogues are known to the skilled person and include derivatives, wherein a sugar is modified, as in 2'-O-methyl, 2'-deoxy-2'-fluoro, and 2',3'-dideoxynucleoside derivatives, nucleic acid analogs based on other sugar backbones, such as threose, locked nucleic acids (LNA), LNA derivatives,

peptide nucleic acids (PNA), glycol nucleic acid (GNA), threose nucleic acid (TNA), bicyclo sugars, or hexose, glycerol and glycol sugars, nucleic acid analogs based on non-ionic backbones, or nucleic acids and their analogs in nonlinear topologies, such as dendrimers, comb-structures, and nanostructures.

The primers may also be linked to various tags (e.g. fluorescent tags, functionalized tags or binding tags), which can optionally be bound to their ends, sugars, or nucleobases.

Primers can be prepared by a variety of methods, including - but not limited to - cloning of appropriate sequences and direct chemical synthesis using methods well known in the art [Narang et al., Methods Enzymol. 68:90 (1979); Brown et al., Methods Enzymol. 68:109 (1979)]. Primers can also be obtained from commercial sources.

In one embodiment, the primers can be designed in manner wherein the primers specifically are capable of amplification of the target polynucleotide sequence.

**Measuring an electrochemical property of the solution**

The method of the second aspect of the invention comprises a step of measuring, a change in the current and/or potential of the electrochemical cell.

As outlined above, in an embodiment the method of the first aspect of the invention comprises a step of measuring an electrochemical response of the electrochemical cell, such as a change in the current and/or potential and/or impedance of the electrochemical cell.

Said change is preferably a result of a change in a concentration of a signaling substance from a nucleic acid amplification process, for example $H^+$, for example a LAMP amplification product or by-product (e.g., $H^+$), or a change in a physical quantity as a result of said LAMP amplification. In particular, the change in $H^+$ results in a change in the oxidation state a quinone, a quinone derivative, and/or a pH indicator which is dissolved in the electrochemical cell, which is measured as change in electrochemical response of the electrochemical cell. Measurement of the change in the electrochemical response (e.g., current and/or potential and/or impedance) of the electrochemical cell of the electrochemical device preferably takes place following contacting the electrochemical device/two or three-electrode electrochemical cell with a measurement setup, for forming an electrochemical system.

The electrochemical system comprises typically the electrochemical device/ three-electrode electrochemical cell, including, where present, the film electrodes, the solution and a separate current path, typically a potentiostat. The electrochemical system thereby comprises multiple film electrodes, such as a working electrode, a counter electrode and a reference electrode, spatially separated and distributed throughout one or more ionically-conductive media, or electrolytes, while also being in electrical contact with one another via a separate current path (preferably comprising, or consisting of, a potentiostat).

The electrodes in an electrochemical system undergo oxidation and reduction reactions, with movement of electrons producing current traveling through the current path simultaneously with movement of ions through the media producing an overall balance of charge transfer within the system.

A potentiostat is typically an instrument designed to control the electrodes in an electrochemical system by adjusting the potential (or current) and measuring the subsequent effect on current (or potential). This task is typically accomplished through a variety of internal circuits, operational amplifiers, and feedback loops. External cables are commonly used to physically connect to each electrode, which typically includes a working electrode, a counter electrode, and a reference electrode.

The measurements may be performed according to any electroanalytical method, for example open circuit potential, potentiometric, impedimetric, coulometric, voltametric and/or amperometric measurements. Typically a measurement of a solution may take between 1 and 90 minutes, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71,72, 73, 74, 75, 76, 77, 78, 79, 80, 81, 82, 83, 84, 85, 86, 87, 88, 89 or 90 minutes.

In one embodiment, the measurement of a solution may take within the range of 1 to 45 minutes, such as 1 to 30 minutes such as 1 to 15 minutes, such as 5 to 10 minutes.

Voltammetry is the study of current as a function of applied potential. Thereby, voltammograms I=f(E) curves are produced. Typically in such a measurement, the potential is varied arbitrarily either step by step or continuously, and the actual current value is measured as the dependent variable. The opposite is amperometry. The shape of the curves depends on the speed of potential variation (nature of driving force) and on whether the solution is stirred or quiescent (mass transfer).

For quantification, analysis requires the consideration of kinetics in addition to thermodynamics, due to the temporal component of voltammetry. Idealized theoretical electrochemical thermodynamic relationships such as the Nernst equation are modeled without a time component. While these models are insufficient alone to describe the dynamic aspects of voltammetry, models like the Tafel equation and Butler-Volmer equation lay the groundwork for the modified voltammetry relationships that relate theory to observed results.

**A particular method of quantifying a target polynucleotide in a sample using LAMP**

In one embodiment there is provided a method of quantifying a target polynucleotide in a sample, said method comprising the steps of:

a. Providing a sample;
b. Applying the sample to an electrochemical device comprising an electrochemical cell having multiple film electrodes;
c. Performing, if the target polynucleotide is present in the sample, a plurality of isothermal LAMP amplifications, each comprising the sample, at least four primers each flanking the target polynucleotide and LAMP reagents comprising a signaling substance and/or capable of releasing a signaling substance, wherein each LAMP amplification releases or consumes the signaling substance;
d. Measuring, simultaneously as step e., a change in the current and/or potential of the electrochemical cell due to a concentration change of the signaling substance; and
e. Deriving an amplification rate, by the rate of the change of the current and/or potential, thereby quantifying the target polynucleotide in the sample.

The following embodiments are not claimed.
In an embodiment the sample is mixed with the primers and LAMP reagents prior to applying the sample to the electrochemical device.
In another embodiment the electrochemical device comprises the primers and the LAMP reagents.
In a further embodiment the multiple film electrodes comprise a working electrode, a reference electrode and a counter electrode.
In an embodiment the working electrode comprises an ion selective membrane, located such that it excludes selected ions of the solution from contacting the working electrode.
In another embodiment the surface of the working electrode is chemically modified to allow for binding of $OH^-$ and $H_3O^+$ ions or is reactive to the pH of the solution.
In an embodiment the surface of any of the electrodes comprises, or is chemically modified by, ruthenium oxide, graphene platelets, or chemically modified such that the electrodes have exposed thiol /hydroxyl groups.
In another embodiment the material of the reference electrode and the counter electrode is Ag/AgCl at around 60/40 w/w ratio.
In a further embodiment the material of the working electrode is carbon.
In an embodiment the electrodes comprise or consist of gold, silver, carbon, platinum, ruthenium dioxide, or a combination thereof.
In another embodiment the material of the electrodes are identical or different.
In a further embodiment the film electrodes are screen printed electrodes.
In a further embodiment the film electrodes are wire electrodes.
In an embodiment the electrochemical device comprises a substrate onto which the electrodes are located, and wherein the material of said substrate is a chemically inert material, such as alumina, ceramic, glass, or an inert plastic.
In another embodiment the electrochemical device comprise a microfluidic inlet, in fluidic connection with the electrochemical cell, for receiving the sample.
In a further embodiment following application of the solution, a vapour barrier is added for preventing evaporation of the solution. In an embodiment the vapor barrier is formed by fluidly sealing the inlet of the electrochemical device, or the microwell, with a physical lid, a heated lid, a mineral oil, and/or a paraffin wax.
In another embodiment a hydrophilic area connects the microfluidic inlet and the electrochemical cell, such as for capillary filling of the electrochemical device.
In a further embodiment the measurements are performed by using a potentiostat or similar circuit.
In an embodiment the measurements comprise or consist of, potentiometric, impedimetric, coulometric, voltammetric and/or amperometric measurements.
In another embodiment the solution is measured between 1 and 90 minutes.
In a further embodiment the LAMP reagents comprise a reverse transcriptase.
In an embodiment the nucleotide amplification is performed at a constant temperature in the range of 59-75°C, such as 62-73°C, such as 64-70°C, such as 66-68°C °C.
In another embodiment the primers comprise a forward inner primer, a backward inner primer, a forward outer primer and a backward outer primer.
In a further embodiment the LAMP amplification is performed with an additional pair of loop primers, such as forward loop primer and/or backward loop primer. Another embodiment relates to a method of determining the presence of a target polynucleotide in a sample. The method comprises discrimination between a target polynucleotide positive sample

and a target polynucleotide negative sample by quantifying and/or determining the polynucleotide amplification rate.

**Sequences**

SEQ ID NO: 1 *ORF1 fragment* of *SARS-CoV-2*

CCCTATGTGTTCATCAAACGTTCGGATGCTCGAACTGCACCTCATGGTCATGTTAT
GGTTGAGCTGGTAGCAGAACTCGAAGGCATTCAGTACGGTCGTAGTGGTGAGAC
ACTTGGTGTCCTTGTCCCTCATGTGGGCGAAATACCAGTGGCTTACCGCAAGGTT
CTTCTTCGTAAGAACGGTAATAAAGGAGCTGGTGGCCATAGTTACGGCGCCGATC
TAAAGTCATTTGACTTAGGCGACGAGCTTGGCACTGATCCTTATGAAGA

SEQ ID NO:2 *Gene N fragment* of *SARS-CoV-2*

ATGACCAAATTGGCTACTACCGAAGAGCTACCAGACGAATTCGTGGTGGTGACGG
TAAAATGAAAGATCTCAGTCCAAGATGGTATTTCTACTACCTAGGAACTGGGCCAG
AAGCTGGACTTCCCTATGGTGCTAACAAAGACGGCATCATATGGGTTGCAACTGA
GGGAGCCTTGAATACACCAAAAGATCACATTGGCACCCGCAATCCTGCTAACAAT
GCTGCAATCGTGCTAC

SEQ ID NO:3 *Forward Inner Primer (FIP)*
AGGTGAGGGTTTTCTACATCACTATATTGGAACAAGCAAATTCTATGG

SEQ ID NO:4 *Backward Inner Primer (BIP)*
ATGGGTTGGGATTATCCTAAATGTGTGCGAGCAAGAACAAGTG

SEQ ID NO:5 *Forward Outer Primer (F3)*
CCACTAGAGGAGCTACTGTA

SEQ ID NO:6 *Backward Outer Primer (B3)*
TGACAAGCTACAACACGT

SEQ ID NO:7 *Forward Loop Primer (FL)*
CAGTTTTTAACATGTTGTGCCAACC

SEQ ID NO:8 *Backward Loop Primer (BL)*
TAGAGCCATGCCTAACATGCT

**Examples**

**Example 1**

<u>Quantitative electroanalytical measurements of a nasal swab sample by LAMP amplification</u>

*Materials and Methods*

<u>Screen printed electrode</u>

i. A screen printed pH sensitive 3-electrode system is used, the system comprising 3 screen printed electrodes which serve as working electrode, reference electrode and auxiliary electrode. The system is printed on chemically inert alumina.

1. The material of the reference electrode is Ag/AgCl (60/40 ratio), with an additional layer providing a fixed concentration of chloride ions for a true reference electrode measurement.

2. The material of the working electrode is Carbon, with pH sensing layer of a solid state membrane with combination of Cobalt Oxide and Iridium Oxide.

3. The material of the counter electrode is Ag/AgCl electrode (60/40 ratio), with an additional layer providing a fixed concentration of chloride ions for a true reference electrode measurement.

ii. Above the electrodes is superimposed a microfluidic sample port in the form of a microwell

iii. A vapor barrier for the microwell is used, in the form of a physical lid.

Primers

A 10X Primer Mix containing 16 $\mu$M FIP, 16 $\mu$M BIP, 2 $\mu$M F3, 2 $\mu$M B3, 4 $\mu$M LoopF, 4 $\mu$M LoopB in nuclease free water (not TE). Final concentration in the amplification reaction mix before the reaction commences are 1.6 $\mu$M FIP, 1.6 $\mu$M BIP, 0.2 $\mu$M F3, 0.2 $\mu$M B3, 0.4 $\mu$M LoopF, 0.4 $\mu$M LoopB.

Sample

i. A nasal swab sample inoculated in the Viral Transport Medium is used after heat treatment at 80°C for 1 minute. An example of the Viral Transport Medium used is Sterile Hanks Balanced Salt Solution (HBSS) 1X with calcium and magnesium ions, with or without Phenol Red, 2% v/v Fetal Bovine Serum, 100$\mu$g/mL Gentamicin sulfate and 0.5$\mu$g/mL for Amphotericin B.

Amplification Reaction

i. 2 uL sample is mixed with

a. 2.5 uL of 25X LAMP Primer Mix targeting the genomic area of interest (final concentration of 1.6 uM FIP, 1.6 uM BIP, 0.2 uM F3, 0.2 uM B3, 0.4 uM LOOP F, 0.4 uM LOOP B),

b. 12.5 uL of WarmStart Colorimetric LAMP 2X Master Mix from New England Biolabs Inc., and

c. Nuclease free water to a final volume of 25 uL.

Amplification Conditions and Assay Design

1. The amplification reaction mixture (with the sample) is added immediately after mixing on the pH sensor preheated at 65°C, already connected to a potentiostat.

2. The vapor barrier is put into place to avoid evaporation.

3. Potentiometric measurements are run realtime and the voltage is measured continuously for 30 minutes.

Interpretation

i.The presence of the nucleic acid template is indicated during the 30 minutes by a drop in pH (pH sensing). The time differential of the change is correlated with quantity of the nucleic acid template.

**Example 2**

Continuous electrochemical real time quantitative monitoring of Loop Mediated

Isothermal Amplification in low Tris buffer using screen printed electrodes.

*Materials and Methods*

Screen printed electrode

i. A screen printed pH sensitive 3-electrode system comprising of 3 screen printed electrodes which serve as working electrode, reference electrode and auxiliary electrode, printed on a chemically inert substrate like alumina or ceramic or glass or inert plastic substrates.
ii. The electrodes may be made of same or different materials like gold, silver, carbon, platinum, ruthenium dioxide, etc.
iii. The working electrode may incorporate a layer of ion selective membrane, and/or the electrode surface may be modified to allow binding of $OH^-$ and $H_3O^+$ ions or is sensitive to pH of the solution when exposed to the solution via direct contact. Such modifications may comprise of Ruthenium oxide, graphene platelets, exposed thiol groups, exposed hydroxyl groups, etc.
iv. Setup:

1. The reference electrode comprises of a Ag/AgCl electrode (60/40 ratio), with an (optional) additional layer providing a fixed concentration of chloride ions for a true reference electrode measurement.

2. The Working electrode comprises of Carbon

3. The counter electrode comprises of a Ag/AgCl electrode (60/40 ratio), with an (optional) additional layer providing a fixed concentration of chloride ions for a true reference electrode measurement.

v. Above the electrodes is superimposed a microfluidic sample port in the form of a microwell (preferred) or other sample introduction/containment methods.
vi. A vapor barrier for the microwell may be used. Alternatives may include physical lid, heated lid, mineral oil vapor barriers, paraffin wax vapor barriers.

Primers

A 10X Primer Mix containing 16 μM FIP, 16 μM BIP, 2 μM F3, 2 μM B3, 4 μM LoopF, 4 μM LoopB in nuclease free water (not TE). Final concentration in the amplification reaction mix before the reaction commences are 1.6 μM FIP, 1.6 μM BIP, 0.2 μM F3, 0.2 μM B3, 0.4 μM LoopF, 0.4 μM LoopB

Samples

ii. A nasal or throat swab sample inoculated in the Viral Transport Medium may be used directly or after heat treatment at 70-90°C for 1 minute.
iii. An anal or vaginal or ear drainage or skin surface swab sample inoculated in the Viral Transport Medium may be used directly or after heat treatment at 70-90°C for 1 minute.
iv. A urine sample may be used may be used directly or after heat treatment at 70-90°C for 1 minute.
v. A whole blood sample mixed with an anticoagulant like heparin, EDTA or sodium citrate, with optional additional

lysis via mechanical lysis, thermal lysis, acoustic cavitation, osmotic shock, enzymatic lysis or chemical lysis.
vi. A lymph drainage sample or a serum sample may be used directly or after heat treatment at 70-90°C for 1 minute.
vii. Purified nucleic acid content from a biological sample or a crude nucleic acid extract

Amplification Reaction

i. Typical amplification reactions contain primers (e.g. four, five or six primers) , sample (which may or may not contain template to which the primers bind ) , nucleotides ( corresponding to G , A , T and C), a buffering agent ( 1 mM to 5 mM Tris or 1 . 5 mM to 5 mM Tris or an equivalent buffer thereof ) , one or more salts (e . g . , ( NH ) 2SO4 , NaCl, MgSO4 , MgCl2 , etc . ) , a bacterial or archaebacterial polymerase (which may or may not be thermostable and may or may not have strand displacing activity ) , and any necessary cofactors and optional detergents , etc. Examples of thermostable polymerases that can be used in a PCR or polymerases for use in isothermal amplification reactions include , but are not limited to Taq , Tfi , Tzi , Tth , Pwo , Pfu , Q5® , Phusion® , One Taq® , Vent® , Deep Vent® , Klenow ( exo - ) , Bst 2 . 0 and Bst 3 . 0 ( New England Biolabs , Ipswich , Mass . ) , PyroPhage® ( Lucigen , Middleton , Wis . ) , Tin DNA polymerase , GspSSD LF DNA polymerase , Rsp ( OptiGene , Horsham , UK) and phi29 polymerase , etc .
ii. Preferred reaction mixture: 2 uL sample (containing DNA/RNA or No-template control; range: 1-5 uL; more than 2 uL sample if a Transport medium is being used inhibits reaction; less than 2 uL will mean there might be too little template) is mixed with

a. 2.5 uL of 25X LAMP Primer Mix targeting the genomic area of interest (final concentration of 1.6 uM FIP, 1.6 uM BIP, 0.2 uM F3, 0.2 uM B3, 0.4 uM LOOP F, 0.4 uM LOOP B),

b. 12.5 uL of WarmStart Colorimetric LAMP 2X Master Mix from New England Biolabs Inc. (also contains reverse transcriptase), and

c. Nuclease free water to a final volume of 25 uL.

iii. Alternative reaction mixture:
2 uL sample (containing DNA/RNA or No-template control; can be up to 5 uL) is mixed with

a. 2.5 uL of 25X LAMP Primer Mix targeting the genomic area of interest (final concentration of 1.6 uM FIP, 1.6 uM BIP, 0.2 uM F3, 0.2 uM B3, 0.4 uM LOOP F, 0.4 uM LOOP B),

b. 12.5 uL of WarmStart LAMP 2X Master Mix from New England Biolabs Inc., and

c. Nuclease free water to a final volume of 25 uL.

Amplification Conditions and Assay Design

i. Preferred

1. The amplification reaction mixture (with the sample) is added immediately after mixing on the pH sensor pre-heated at 59-75°C, already connected to a potentiostat or equivalent circuit.

2. The vapor barrier is put into place to avoid evaporation.

3. Potentiometric measurements are run realtime and the voltage is measured continuously for 1-90 minutes.

4. The assay can be parallelized for multiple samples.

ii. Alternative

1. The mixing of the sample with the rest of the components may happen directly on the chip using a pipette or

microfluidic mixing.

2. The amplification reaction mixture (with the sample) is added in a PCR or microfuge tube and heated at 59-75°C. The sample is aliquoted after 1-90 minutes, or at intervals of 1-5 minutes 2-5 times, and the pH is measured on the pH sensor at room temperature already connected to a potentiostat or equivalent circuit.

3. Instead of pH, impedimetric measurements, coulometric measurements, amperometric measurements may be performed without the use of any additional redox probe or DNA intercalating dye.

Interpretation

i.The presence of the nucleic acid template is indicated between 1-90 minutes by

i. A drop in pH (pH sensing)

ii. A drop in conductivity (amperometry)

iii. Increase in impedance (impedimetry)

iv. Change in net charge (coulometry)

ii. Further, the time differential of this change is correlated with quantity of the nucleic acid template

**Example 3**

A three-electrode system method for monitoring the pH of a solution containing phenol red

Parameters Used

In this example, 50μL of an aqueous solution containing PBS, NaOH or HCl (for pH adjust), 0.1 M KCl as supporting electrolyte and 100uM phenol red as pH probe was analysed.

The aqueous solution was introduced into a three-electrode electrochemical cell and squarewave voltammetry was performed on the electrochemical cell with the following parameters:

a. Peak at 0.5 V vs. Ag/AgCl. Scan range 0 to 0.6 V vs. Ag/AgCl; frequency: 10 Hz; potential step: 5mV; Amplitute: 10mV.

b. Peak at between -0.4 to -0.55 V vs. Ag/AgCl. Scan range 0 to -0.7 V vs. Ag/AgCl; frequency: 10 Hz; potential step: 5mV; Amplitute: 10mV.

$N_20_3$-Au disposable electrodes were used along with an Anapot potentiostat.

The pH change was analysed as a function of peak height (i) and peak position which is defined by the potential (E):

$$\Delta pH = f(i^*E) + f(i) + f(E)$$

Results of Analysis

Phenol red is a tri-quinone structured chemical, which undergoes three-electron transfer in the potential window of -1V to 1V (vs Ag/AgCl, see Figure 3 for an overview of redox peaks for phenol red). The peak current and peak potential are both pH dependent. Despite its limited visible light spectrum between pH 6 - pH 8, the redox potentials of phenol red and the corresponding peak currents are capable of the full pH range identification.

Phenol red is the simplest form of the sulfonephthalein group and a weak acid with a pKa of 7.9 and is one of the most commonly used pH indicators. Its chemical structure and the dissociation in aqueous solution can be seen in the following scheme:

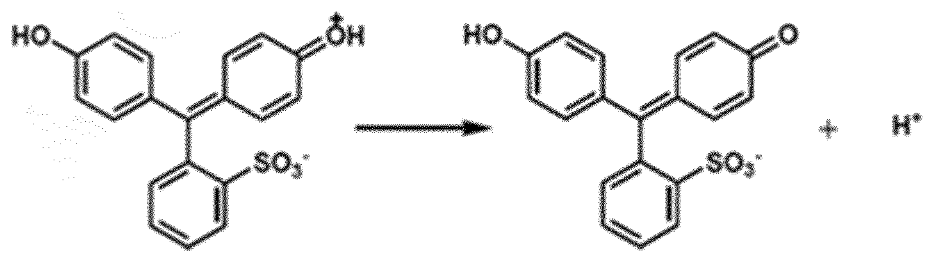

[A] [B]

The ionizable sector of quinone methide in the tri-aromatic structure of phenol red imparts rich redox properties which shows advantageous characteristics for various applications. The sector of quinone methide in the structure is the center of the rich redox chemistry. Cyclic voltammograms of phenol red scanned at varying scan rates with a bare GC electrode in solutions buffered at pH 7.9 in PBS are shown in Figure 4. Six significant waves can be observed in Figure 4. By correlating peak currents with the values of scan rate and square root of scan rate, as shown in the inset plot of Figure 4, it was found that the waves (b), (c), (d), and (f) can be categorized as redox reactions of phenol red at the electrode interface, since the fact that peak current showing proportional to square root of scan rate is characteristic of diffusion-controlled reactions. In contrast, the waves (a) and (e) are categorized as adsorption process of phenol red on the electrode surface, due to the linear relation of peak current with the scan rate. In order to identify the redox couples of phenol red, cyclic voltammetry scans of phenol red were conducted at varying switching potentials, the results of which are shown in Figure 5. The scans were initiated cathodically from 1.5 V to switching potential of 0.4, -0.2, -1.0, and -1.5 V, respectively. Looking to the far right of the curves in the inset graph in Figure 5, curve (a) shows two reduction peaks with only one significant oxidation peak whereas curve (b) shows simply one redox couple. The ipc/ipa ratio for curve (a) is about 2.8 and that of curve (b) is 2.3, indicating a decline of reduced species in the process of curve (a).

The following two-step reduction may account for the finding:

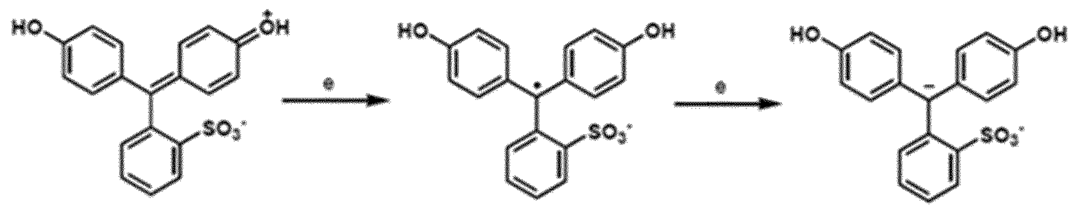

[A] [C] [D]

The peak of the first reduction took place at -0.75 V followed by the second reduction situated at -1.12 V in curve (a) of the inset graph in Figure 5. Moreover, a disproportionation consumes a fraction of the free radical product of the first reduction [C] into a carbanion [D] and the starting oxonium ion [A]:

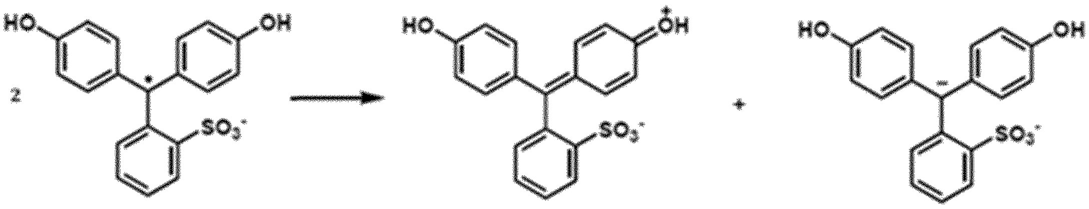

[C] [A] [D]

This causes the decline of both oxidation peaks in curves (a) and (b) and hence the deviation of $i_{pc}/i_{pa}$ ratio from a general reversible process. In addition, because the protonation of the carbanion species is an irreversible reaction, the second reduction peak at -1.12 V is much smaller compared with that of the first counterpart at -0.75 V. Based on the aforementioned, it accounts for the relatively lower $i_{pc}/i_{pa}$ in curve (a) compared to that of curve (b). The influence of pH value on the reductions and oxidations of phenol red are illustrated in Figure 6, solution of pH 3.9 (curves (a) and (a'), [A] being the main species), 7.9 (curves (b) and (b'), equal in amount of both forms), and 11.9 (curves (c) and (c'), mainly [B]) containing phenol red were buffered with PBS and CV scans of these solutions were conducted both in anodic and cathodic directions. It is worth noting that, due to the dependence of peak potential on pH variation, the first reductions of (a') to (c') include one electron and one proton transfer while the second reduction proceeds without proton

transfer.

The corresponding anodic scans of the solutions (a) to (c) exhibit two oxidation peaks for curves (a) and (b) despite only one for (c). For the cases of (a) and (b), the unprotonated phenol red [B] undergoes first oxidation and followed by another oxidation of the protonated counterpart [A] at a more positive potential accompanied by deprotonation. In order to clarify the connection between both oxidations, a scan started before the potential of second oxidation (0.90 V) was further conducted as shown in curve (d). The result suggests that the second oxidation is not derived from the product of the first oxidation. By contrast, the absence of the second oxidation in curve (c) is owing to a shortfall of [A] in an alkaline environment. Therefore, the oxidations from both species of phenol red result in a radical cation [E], as revealed by the following routes:

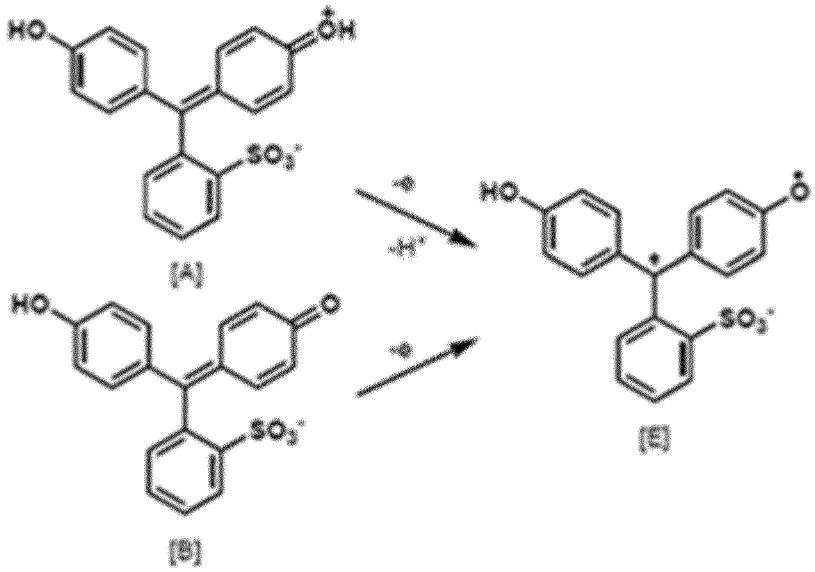


Squarewave voltammetry is used for the signal identification, benefiting from excellent signal enhancement as compared to cyclic voltammetry, facilitating simple signal processing. The optimal concentration of Phenol Red was observed to be around 100 uM. Without wishing to be bound by theory, 100μM gives well defined redox peak without inhibiting nuclei amplification. However, a wide range of phenol red concentrations may be used with signal identification still being achieved.

The results of performing squarewave voltammetry on phenol red in the pH range of 6.5 to 8.5, at different concentrations of phenol red, may be seen in Figure 7. The lower two graphs of Figure 7 show peak positions (voltage) and heights (current) versus concentrations of phenol red.

A linear regression algorithm was then applied to obtain an accurate value of pH, i.e. the output signal obtained was correlated to the pH value by application of a linear regression algorithm. This algorithm was validated with fitting the predicted value with actual value, with an error within 0.05 pH, the results of which can be seen in Figure 8.

Besides the peaks in the scan between 0-800 mV, two other peaks were also observed (see Figure 9).

Peaks a) b) and c) as highlighted in Figure 9 are pH dependent. This study focused on peaks a) and b), as peak c) is too close to hydrolysis, which is irreversible EC peak. Peak a): From high pH to low pH: shifts to positive potential; 1 electron transfer across pH range (~50-60mV/pH). Peak b): From high pH to low pH: shifts to negative potential; number of electron transfer depends on pH (~30-60mV/pH).

The extreme accuracy of pH quantification that this method provides despite the very small sample size required has many applications, including highly accelerated monitoring and quantification of nucleic acid amplification from small samples as shown in Example 4.

## Claims

**1.** A method of measuring the pH of a solution, wherein the method comprises the steps of:

- providing a solution comprising a quinone, a quinone derivative, and/or a pH indicator, wherein the quinone, quinone derivative, and/or pH indicator is dissolved in the solution;
- applying the solution to a three-electrode electrochemical cell;
- measuring an electrochemical response of the electrochemical cell; and
- quantifying the pH of the solution as a function of the electrochemical response of the electrochemical cell, wherein the response correlates with the electrochemical state of the quinone, quinone derivative and/or pH indicator.

**2.** The method according to Claim 1, wherein the quinone or quinone derivative is selected from the list consisting of 1,2-benzoquinone, 1,4-benzoquinone, 1,4-naphthoquinone, 9,10-anthraquinone, and derivatives and combinations thereof, and
wherein the pH indicator is selected from the list consisting of malachite green oxalate, brilliant green, eosin yellowish, erythrosine B, methyl green, methyl violet, picric acid, cresol red, crystal violet, m-Cresol purple, thymol blue, p-Xylenol blue, Eosin (bluish), quinaldine red, 2,4-dinitro phenol, 4-(dimethylamino) azobenzol, bromochlorophenol blue, bromophenol blue, congo red, methyl orange, bromocresol green, 2,5-dinitrophenol, alizarin sulphonic acid, methyl red, chlorophenol red, litmus, bromocresol purple, bromophenol red, 4-nitrophenol, bromoxylenol blue, bromothymol blue, phenol red, 3-nitrophenol, neutral red, creosol red, 1-naphtholphthalein, m-cresol purple, thymol blue, p-xylenol blue, phenolphthalein, thymolphthalein, alkali blue, alizarin yellow GG, indigo carmine, epsilon blue, titan yellow, and combinations thereof.

**3.** The method according to any one of the preceding claims, wherein the electrochemical response of the electrochemical cell that is measured is the potential of the electrochemical cell, the current of the electrochemical cell, the impedance of the electrochemical cell, or a combination of these, such as wherein the electrochemical response of the electrochemical cell that is measured comprises a combination of the potential of the electrochemical cell, the current of the electrochemical cell, the impedance of the electrochemical cell.

**4.** The method according to any one of the preceding claims, wherein the potential of the electrochemical cell is measured via cyclic squarewave voltammetry, squarewave voltammetry, linear sweep voltammetry, cyclic voltammetry or open circuit potentiometry; wherein the step of quantifying the pH of the solution as a function of the electrochemical response of the electrochemical cell is performed via application of a regression algorithm..

**5.** The method according to any one of the preceding claims, wherein the concentration of the quinone, a quinone derivative, and/or a pH indicator within the solution is from about 1 $\mu$M to about 1000 $\mu$M, such as from about 1 $\mu$M to about 500 $\mu$M, for example from about 5 $\mu$M to about 250 $\mu$M, in particular from about 50 $\mu$M to about 200 $\mu$M.

**6.** The method according to any preceding claim, wherein the solution comprises a buffer, and wherein the solution further comprises:

a. a sample, preferably wherein the sample is selected from the list consisting of a nasal sample, a throat sample, an anal sample, a vaginal sample, an ear draining sample, a skin surface swab sample, a urine sample, a whole blood sample, a serum sample, a plasma sample and a lymph drainage sample;
b. primers and nucleic acid amplification reagents, preferably wherein the nucleic acid amplification reagents are LAMP reagents.

**7.** The method according to claim 6, wherein the method comprises a step of performing a nucleic acid amplification step, preferably a plurality of nucleic acid amplification steps, such as wherein the nucleic acid amplification step(s) is/are a loop-mediated isothermal amplification (LAMP) step, and wherein the method is for determining the presence of a target polynucleotide in the solution; and wherein the nucleotide amplification is performed at a constant temperature in the range of from 59 to 75°C, such as from 62 to 73°C, such as from 64 to 70°C, such as from 66 to 68°C.

**8.** The method according to any one of the preceding claims, wherein said method quantifies a change in pH of the solution.

**9.** The method according to any of the preceding claims, wherein the three-electrode electrochemical cell comprises a working electrode, a reference electrode and a counter electrode; and

wherein the electrodes comprise or consist of gold, silver, carbon, platinum, ruthenium dioxide, or a combination thereof;
wherein the material of the electrodes are identical or different, and
wherein the electrodes of the three-electrode electrochemical cell are film electrodes, such as screen-printed electrodes, or wire electrodes.

**10.** The method according to any one of the preceding claims, wherein the measurements are performed by using a potentiostat or similar circuit; and wherein the measurements are performed for a period of 1 minute to 90 minutes.

**11.** Use of a three-electrode electrochemical cell for monitoring a nucleic acid amplification reaction, such as loop-mediated isothermal amplification (LAMP), said use comprising:

- providing a solution comprising a quinone, a quinone derivative, a pH indicator, or combinations thereof, wherein the quinone, quinone derivative, and/or pH indicator is dissolved in the solution;
- providing a LAMP mixture comprising: a sample comprising a target polynucleotide, at least two, such as at least four primers each flanking the target polynucleotide and LAMP reagents;
- applying the solution and the LAMP mixture to the three-electrode electrochemical cell;
- measuring an electrochemical response of the electrochemical cell; and
- quantifying the pH of the solution as a function of the electrochemical response of the electrochemical cell, wherein the response correlates with the electrochemical state of the quinone, quinone derivative and/or pH indicator.

**12.** Use of a system comprising:

a. a potentiostat; and
b. a measuring kit, said kit comprising:

i. a first receptacle comprising a three-electrode electrochemical cell, and a second receptacle comprising a quinone, a quinone derivative, and/or a pH indicator, or
ii. a first receptacle comprising a three-electrode electrochemical cell and a quinone, a quinone derivative, and/or a pH indicator,

wherein the quinone, quinone derivative, and/or pH indicator are dissolved in an aqueous solution in the first or in the second receptacle,
for measuring the pH of a solution.

**13.** A system for measuring the pH of a solution, the system comprising:

a. a potentiostat;
b. a measuring kit, said kit comprising:

i. a first receptacle comprising a three-electrode electrochemical cell, and a second receptacle comprising a quinone, a quinone derivative, and/or a pH indicator, or
ii. a first receptacle comprising a three-electrode electrochemical cell and a quinone, a quinone derivative, and/or a pH indicator,
wherein the quinone, quinone derivative, and/or pH indicator are dissolved in an aqueous solution in the first or in the second receptacle, and
wherein said aqueous solution comprises at least four primers configured to flank a target polynucleotide sequence and LAMP reagents; and

c. a heating unit, configured for heating the first receptacle, such as for heating the first receptacle to a temperature within the range of 59°C to 75°C.

**14.** The system according to claim 13, wherein the potentiostat is configured to measure an electrochemical response of the electrochemical cell, wherein the electrochemical response is representative of the oxidation state of the quinone, quinone derivative, and/or pH indicator in the first receptacle.

**15.** The system according to any one of claims 13 and 14, wherein the first receptacle comprises a microfluidic inlet for receiving a solution and/or a sample.

## Patentansprüche

**1.** Verfahren zur Messung des pH-Werts einer Lösung, wobei das Verfahren die folgenden Schritte umfasst:

- Bereitstellen einer Lösung, die ein Chinon, ein Chinonderivat und/oder einen pH-Indikator umfasst, wobei das Chinon, das Chinonderivat und/oder der pH-Indikator in der Lösung gelöst ist;
- Aufbringen der Lösung auf eine elektrochemische Zelle mit drei Elektroden;
- Messen einer elektrochemischen Reaktion der elektrochemischen Zelle; und
- Quantifizieren des pH-Werts der Lösung als Funktion der elektrochemischen Reaktion der elektrochemischen Zelle, wobei die Reaktion mit dem elektrochemischen Zustand des Chinons, des Chinonderivats und/oder des pH-Indikators korreliert.

**2.** Verfahren nach Anspruch 1, wobei das Chinon oder Chinonderivat ausgewählt ist aus der Liste bestehend aus 1,2-Benzochinon, 1,4-Benzochinon, 1,4-Naphthochinon, 9,10-Anthrachinon, und Derivaten und Kombinationen davon, und wobei der pH-Indikator ausgewählt ist aus der Liste bestehend aus Malachitgrünoxalat, Brillantgrün, Eosin gelblich, Erythrosin B, Methylgrün, Methylviolett, Pikrinsäure, Kresolrot, Kristallviolett, m-Kresolpurpur, Thymolblau, p-Xylenolblau, Eosin (bläulich), Chinaldinrot, 2,4-Dinitrophenol, 4-(Dimethylamino)azobenzol, Bromchlorphenolblau, Bromphenolblau, Kongorot, Methylorange, Bromkresolgrün, 2,5-Dinitrophenol, Alizarinsulfonsäure, Methylrot, Chlorphenolrot, Lackmus, Bromkresolpurpur, Bromphenolrot, 4-Nitrophenol, Bromxylenolblau, Bromthymolblau, Phenolrot, 3-Nitrophenol, Neutralrot, Kreosolrot, 1-Naphtholphthalein, m-Kresolpurpur, Thymolblau, p-Xylenolblau, Phenolphthalein, Thymolphthalein, Alkaliblau, Alizaringelb GG, Indigokarmin, Epsilonblau, Titangelb, und Kombinationen davon.

**3.** Verfahren nach einem der vorstehenden Ansprüche, wobei die elektrochemische Reaktion der elektrochemischen Zelle, die gemessen wird, das Potenzial der elektrochemischen Zelle, der Strom der elektrochemischen Zelle, die Impedanz der elektrochemischen Zelle, oder eine Kombination davon, ist, beispielsweise wobei die elektrochemische Reaktion der elektrochemischen Zelle, die gemessen wird, eine Kombination aus dem Potenzial der elektrochemischen Zelle, dem Strom der elektrochemischen Zelle, der Impedanz der elektrochemischen Zelle umfasst.

**4.** Verfahren nach einem der vorstehenden Ansprüche, wobei das Potenzial der elektrochemischen Zelle über zyklische Rechteckwellenvoltammetrie, Rechteckwellenvoltammetrie, lineare Sweep-Voltammetrie, zyklische Voltammetrie oder Leerlaufpotentiometrie gemessen wird; wobei der Schritt des Quantifizierens des pH-Werts der Lösung als eine Funktion der elektrochemischen Reaktion der elektrochemischen Zelle durch Anwendung eines Regressionsalgorithmus durchgeführt wird.

**5.** Verfahren nach einem der vorstehenden Ansprüche, wobei die Konzentration des Chinons, eines Chinonderivats und/oder eines pH-Indikators in der Lösung etwa 1 µM bis etwa 1000 µM, wie etwa 1 µM bis etwa 500 µM, beispielsweise etwa 5 µM bis etwa 250 µM, insbesondere etwa 50 µM bis etwa 200 µM, beträgt.

**6.** Verfahren nach einem der vorstehenden Ansprüche, wobei die Lösung einen Puffer umfasst und wobei die Lösung ferner Folgendes umfasst:

a. eine Probe, wobei die Probe vorzugsweise ausgewählt ist aus der Liste bestehend aus einer Nasenprobe, einer Rachenprobe, einer Analprobe, einer Vaginalprobe, einer Ohrdrainageprobe, einer Hautoberflächenabstrichprobe, einer Urinprobe, einer Vollblutprobe, eine Serumprobe, eine Plasmaprobe und eine Lymphdrainageprobe;
b. Primer und Nukleinsäure-Amplifikationsreagenzien, wobei die Nukleinsäure-Amplifikationsreagenzien vorzugsweise LAMP-Reagenzien sind.

**7.** Verfahren nach Anspruch 6, wobei das Verfahren einen Schritt des Durchführens eines Nukleinsäure-Amplifikationsschritts umfasst, vorzugsweise eine Vielzahl von Nukleinsäure-Amplifikationsschritten, wobei es sich bei dem Nukleinsäure-Amplifikationsschritt bzw. den Nukleinsäure-Amplifikationsschritten beispielsweise um einen LAMP-Schritt (LAMP - loop-mediated isothermal amplification, schleifenvermittelte isothermale Amplifikation) handelt, und wobei das Verfahren zum Bestimmen des Vorhandenseins eines Zielpolynukleotids in der Lösung dient; und wobei die Nukleotid-Amplifikation bei einer konstanten Temperatur im Bereich von 59 bis 75 °C, wie 62 bis 73 °C, wie 64 bis 70 °C, wie 66 bis 68 °C, durchgeführt wird.

**8.** Verfahren nach einem der vorstehenden Ansprüche, wobei das Verfahren eine Änderung des pH-Werts der Lösung quantifiziert.

**9.** Verfahren nach einem der vorstehenden Ansprüche, wobei die elektrochemische Zelle mit drei Elektroden eine Arbeitselektrode, eine Referenzelektrode und eine Gegenelektrode umfasst; und

wobei die Elektroden Gold, Silber, Kohlenstoff, Platin, Rutheniumdioxid, oder eine Kombination davon, umfassen oder daraus bestehen;
wobei das Material der Elektroden gleich oder verschieden ist, und
wobei es sich bei den Elektroden der elektrochemischen Zelle mit drei Elektroden um Filmelektroden, wie Screen Printed-Elektroden oder Drahtelektroden, handelt.

**10.** Verfahren nach einem der vorstehenden Ansprüche, wobei die Messungen unter Verwendung eines Potentiostaten oder einer ähnlichen Schaltung durchgeführt werden; und wobei die Messungen für einen Zeitraum von 1 Minute bis 90 Minuten durchgeführt werden.

**11.** Verwendung einer elektrochemischen Zelle mit drei Elektroden zur Überwachung einer Nukleinsäure-Amplifikationsreaktion, wie einer schleifenvermittelten isothermalen Amplifikation (LAMP), wobei die Verwendung Folgendes umfasst:

- Bereitstellen einer Lösung, die ein Chinon, ein Chinonderivat, einen pH-Indikator, oder Kombinationen davon, umfasst, wobei das Chinon, das Chinonderivat und/oder der pH-Indikator in der Lösung gelöst ist;
- Bereitstellen eines LAMP-Gemischs, das Folgendes umfasst: eine Probe umfassend ein Zielpolynukleotid, mindestens zwei, beispielsweise mindestens vier, Primer, die jeweils das Zielpolynukleotid flankieren, und LAMP-Reagenzien;
- Auftragen der Lösung und des LAMP-Gemischs auf die elektrochemische Zelle mit drei Elektroden;
- Messen einer elektrochemischen Reaktion der elektrochemischen Zelle; und
- Quantifizieren des pH-Werts der Lösung als Funktion der elektrochemischen Reaktion der elektrochemischen Zelle, wobei die Reaktion mit dem elektrochemischen Zustand des Chinons, des Chinonderivats und/oder des pH-Indikators korreliert.

**12.** Verwendung eines Systems, umfassend:

a. einen Potentiostaten; und
b. ein Messkit, wobei das Kit Folgendes umfasst:

i. ein erstes Behältnis, das eine elektrochemische Zelle mit drei Elektroden umfasst, und ein zweites Behältnis, das ein Chinon, ein Chinonderivat und/oder einen pH-Indikator umfasst, oder
ii. ein erstes Behältnis, das eine elektrochemische Zelle mit drei Elektroden und ein Chinon, ein Chinonderivat und/oder einen pH-Indikator umfasst,

wobei das Chinon, das Chinonderivat und/oder der pH-Indikator in einer wässrigen Lösung in dem ersten oder in dem zweiten Behältnis gelöst sind, zur Messung des pH-Wertes einer Lösung.

**13.** System zur Messung des pH-Werts einer Lösung, wobei das System Folgendes umfasst:

a. einen Potentiostaten;
b. ein Messkit, wobei das Kit Folgendes umfasst:

i. ein erstes Behältnis, das eine elektrochemische Zelle mit drei Elektroden umfasst, und ein zweites Behältnis, das ein Chinon, ein Chinonderivat und/oder einen pH-Indikator umfasst, oder
ii. ein erstes Behältnis, das eine elektrochemische Zelle mit drei Elektroden und ein Chinon, ein Chinonderivat und/oder einen pH-Indikator umfasst,
wobei das Chinon, das Chinonderivat und/oder der pH-Indikator in einer wässrigen Lösung in dem ersten oder in dem zweiten Behältnis gelöst sind und
wobei die wässrige Lösung mindestens vier Primer umfasst, die dazu ausgelegt sind, eine Zielpolynukleotidsequenz zu flankieren, und LAMP-Reagenzien; und

c. eine Heizeinheit, die zum Erwärmen des ersten Behältnisses ausgelegt ist, beispielsweise zum Erwärmen des ersten Behältnisses auf eine Temperatur im Bereich von 59 °C bis 75 °C.

**14.** System nach Anspruch 13, wobei der Potentiostat dazu ausgelegt ist, eine elektrochemische Reaktion der elektrochemischen Zelle zu messen, wobei die elektrochemische Reaktion repräsentativ für den Oxidationszustand des Chinons, des Chinonderivats und/oder des pH-Indikators in dem ersten Behältnis ist.

**15.** System nach einem der Ansprüche 13 und 14, wobei das erste Behältnis einen mikrofluidischen Einlass zum Aufnehmen einer Lösung und/oder eine Probe umfasst.

## Revendications

**1.** Procédé de mesure du pH d’une solution, dans lequel le procédé comprend les étapes :

- de fourniture d’une solution comprenant une quinone, un dérivé de quinone et/ou un indicateur de pH, dans lequel la quinone, le dérivé de quinone, et/ou l’indicateur de pH est dissous dans la solution ;
- d’application de la solution à une cellule électrochimique à trois électrodes ;
- de mesure d’une réponse électrochimique de la cellule électrochimique ; et
- de quantification du pH de la solution en fonction de la réponse électrochimique de la cellule électrochimique, dans lequel la réponse est en corrélation avec l’état électrochimique de la quinone, du dérivé de la quinone et/ou de l’indicateur de pH.

**2.** Procédé selon la revendication 1, dans lequel la quinone ou le dérivé de quinone est choisi dans la liste consistant en 1,2-benzoquinone, la 1,4-benzoquinone, la 1,4-naphtoquinone, la 9,10-anthraquinone, et les dérivés et combinaisons de celles-ci, et
dans lequel l’indicateur de pH est choisi dans la liste consistant en oxalate de vert malachite, vert brillant, éosine jaunâtre, érythrosine B, vert de méthyle, violet de méthyle, acide picrique, rouge de crésol, violet cristal, violet de m-crésol, bleu de thymol, bleu de p-xylénol, éosine (bleuâtre), rouge de quinaldine, 2,4-dinitrophénol, 4-(diméthylamino)azobenzol, bleu de bromochlorophénol, bleu de bromophénol, rouge congo, orange de méthyle, vert de bromocrésol, 2,5-dinitrophénol, acide alizarine sulfonique, rouge de méthyle, rouge de chlorophénol, tournesol, violet de bromocrésol, rouge de bromophénol, 4-nitrophénol, bleu de bromoxylénol, bleu de bromothymol, rouge de phénol, 3-nitrophénol, rouge neutre, rouge de créosol, 1-naphtolphtaléine, violet de m-crésol, bleu de thymol, bleu de p-xylénol, phénolphtaléine, thymolphtaléine, bleu alcalin, jaune alizarine GG, carmin d’indigo, bleu epsilon, jaune titan et leurs combinaisons.

**3.** Procédé selon l’une quelconque des revendications précédentes, dans lequel la réponse électrochimique de la cellule électrochimique qui est mesurée est le potentiel de la cellule électrochimique, le courant de la cellule électrochimique, l’impédance de la cellule électrochimique, ou une combinaison de ceux-ci, tels que dans lequel la réponse électrochimique de la cellule électrochimique qui est mesurée comprend une combinaison du potentiel de la cellule électrochimique, du courant de la cellule électrochimique, de l’impédance de la cellule électrochimique.

**4.** Procédé selon l'une quelconque des revendications précédentes, dans lequel le potentiel de la cellule électrochimique est mesuré par voltammétrie cyclique à vagues carrée, voltammétrie à vagues carrée, voltammétrie de balayage linéaire, voltammétrie cyclique ou potentiométrie à circuit ouvert ; dans lequel l'étape de quantification du pH de la solution en fonction de la réponse électrochimique de la cellule électrochimique est réalisée via l'application d'un algorithme de régression.

**5.** Procédé selon l'une quelconque des revendications précédentes, dans lequel la concentration de la quinone, d'un dérivé de la quinone et/ou d'un indicateur de pH à l'intérieur de la solution est d'environ 1 μM à environ 1 000 μM, comme d'environ 1 μM à environ 500 μM, par exemple d'environ 5 μM à environ 250 μM, en particulier d'environ 50 μM à environ 200 μM.

**6.** Procédé selon une quelconque revendication précédente, dans lequel la solution comprend un tampon, et dans lequel la solution comprend en outre :

a. un échantillon, de préférence dans lequel l'échantillon est sélectionné dans la liste consistant en un échantillon nasal, un échantillon de gorge, un échantillon anal, un échantillon vaginal, un échantillon d'écoulement d'oreille, un échantillon de frottis de surface de peau, un échantillon d'urine, un échantillon de sang complet, un échantillon de sérum, un échantillon de plasma et un échantillon de drainage lymphatique ;
b. des amorces et des réactifs d'amplification d'acide nucléique, de préférence dans lequel les réactifs d'amplification d'acide nucléique sont des réactifs LAMP.

**7.** Procédé selon la revendication 6, dans lequel le procédé comprend une étape de réalisation d'une étape d'amplification d'acide nucléique, de préférence une pluralité d'étapes d'amplification d'acide nucléique, telle que dans lequel la ou les étapes d'amplification d'acide nucléique est/sont une étape d'amplification isotherme médiée par boucle (LAMP), et dans lequel le procédé consiste à déterminer la présence d'un polynucléotide cible dans la solution ; et dans lequel l'amplification nucléotidique est réalisée à une température constante dans la plage de 59 à 75 °C, telle que de 62 à 73 °C, telle que de 64 à 70 °C, telle que de 66 à 68 °C.

**8.** Procédé selon l'une quelconque des revendications précédentes, dans lequel ledit procédé quantifie un changement de pH de la solution.

**9.** Procédé selon l'une quelconque des revendications précédentes, dans lequel la cellule électrochimique à trois électrodes comprend une électrode de travail, une électrode de référence et une contre-électrode ; et

dans lequel les électrodes comprennent ou sont constituées d'or, d'argent, de carbone, de platine, de dioxyde de ruthénium ou d'une combinaison de ceux-ci ;
dans lequel les matériaux des électrodes sont identiques ou différents, et
dans lequel les électrodes de la cellule électrochimique à trois électrodes sont des électrodes à film, telles que des électrodes sérigraphiées, ou des électrodes à fil.

**10.** Procédé selon l'une quelconque des revendications précédentes, dans lequel les mesures sont effectuées à l'aide d'un potentiostat ou d'un circuit similaire ; et dans lequel les mesures sont effectuées pendant une période de 1 minute à 90 minutes.

**11.** Utilisation d'une cellule électrochimique à trois électrodes pour surveiller une réaction d'amplification d'acide nucléique, telle qu'une amplification isotherme médiée par boucle (LAMP), ladite utilisation comprenant :

- la fourniture d'une solution comprenant une quinone, un dérivé de quinone, un indicateur de pH, ou des combinaisons de ceux-ci, dans lequel la quinone, le dérivé de quinone et/ou l'indicateur de pH est dissous dans la solution ;
- la fourniture d'un mélange LAMP comprenant : un échantillon comprenant un polynucléotide cible, au moins deux, tel qu'au moins quatre amorces flanquant chacune le polynucléotide cible et des réactifs LAMP ;
- l'application de la solution et du mélange LAMP à la cellule électrochimique à trois électrodes ;
- la mesure d'une réponse électrochimique de la cellule électrochimique ; et
- la quantification du pH de la solution en fonction de la réponse électrochimique de la cellule électrochimique, dans lequel la réponse est en corrélation avec l'état électrochimique de la quinone, du dérivé de la quinone et/ou de l'indicateur de pH.

**12.** Utilisation d'un système comprenant :

a. un potentiostat ; et
b. un kit de mesure, ledit kit comprenant :

i. un premier réceptacle comprenant une cellule électrochimique à trois électrodes, et un second réceptacle comprenant une quinone, un dérivé de quinone et/ou un indicateur de pH, ou
ii. un premier réceptacle comprenant une cellule électrochimique à trois électrodes et une quinone, un dérivé de quinone et/ou un indicateur de pH,

dans lequel la quinone, le dérivé de quinone et/ou l'indicateur de pH sont dissous dans une solution aqueuse dans le premier ou dans le second réceptacle, pour mesurer le pH d'une solution.

**13.** Système pour mesurer le pH d'une solution, le système comprenant :

a. un potentiostat ;
b. un kit de mesure, ledit kit comprenant :

i. un premier réceptacle comprenant une cellule électrochimique à trois électrodes, et un second réceptacle comprenant une quinone, un dérivé de quinone et/ou un indicateur de pH, ou
ii. un premier réceptacle comprenant une cellule électrochimique à trois électrodes et une quinone, un dérivé de quinone et/ou un indicateur de pH,
dans lequel la quinone, le dérivé de quinone et/ou l'indicateur de pH sont dissous dans une solution aqueuse dans le premier ou dans le second réceptacle, et
dans lequel ladite solution aqueuse comprend au moins quatre amorces configurées pour flanquer une séquence polynucléotidique cible et des réactifs LAMP ; et

c. une unité de chauffage, configurée pour chauffer le premier réceptacle, par exemple pour chauffer le premier réceptacle à une température dans la plage de 59 °C à 75 °C.

**14.** Système selon la revendication 13, dans lequel le potentiostat est configuré pour mesurer une réponse électrochimique de la cellule électrochimique, dans lequel la réponse électrochimique est représentative de l'état d'oxydation de la quinone, du dérivé de la quinone, et/ou de l'indicateur de pH dans le premier réceptacle.

**15.** Système selon l'une quelconque des revendications 13 et 14, dans lequel le premier réceptacle comprend une entrée microfluidique pour recevoir une solution et/ou un échantillon.

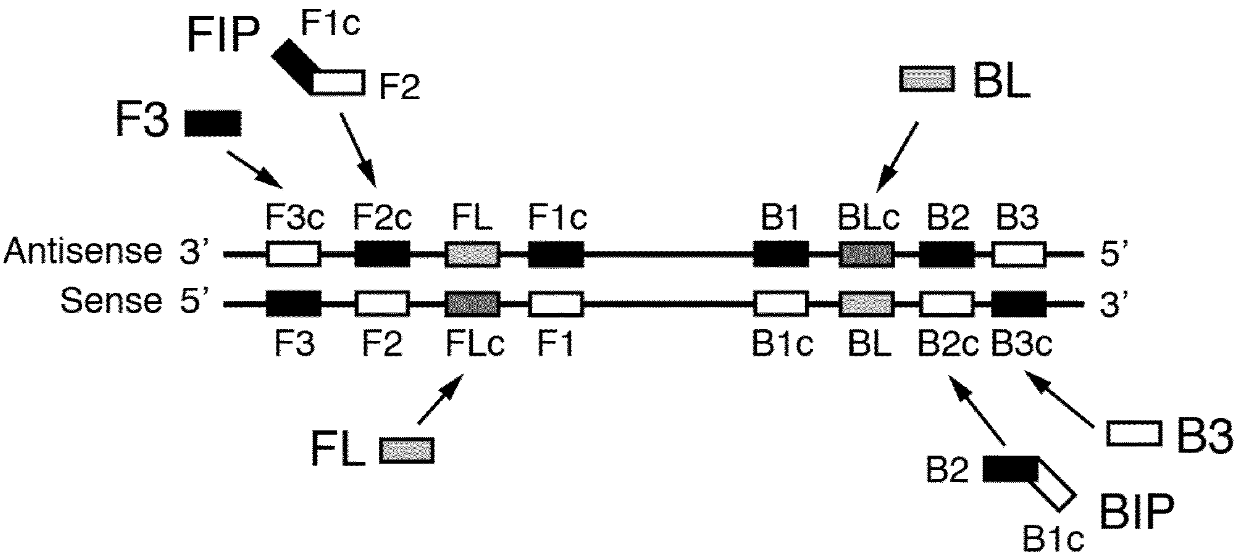
FIP
F1c
F2
F3
BL
F3c
F2c
FL
F1c
B1
BLc
B2
B3
Antisense 3'
5'
Sense 5'
3'
F3
F2
FLc
F1
B1c
BL
B2c
B3c
FL
B3
B2
BIP
B1c

Fig. 1

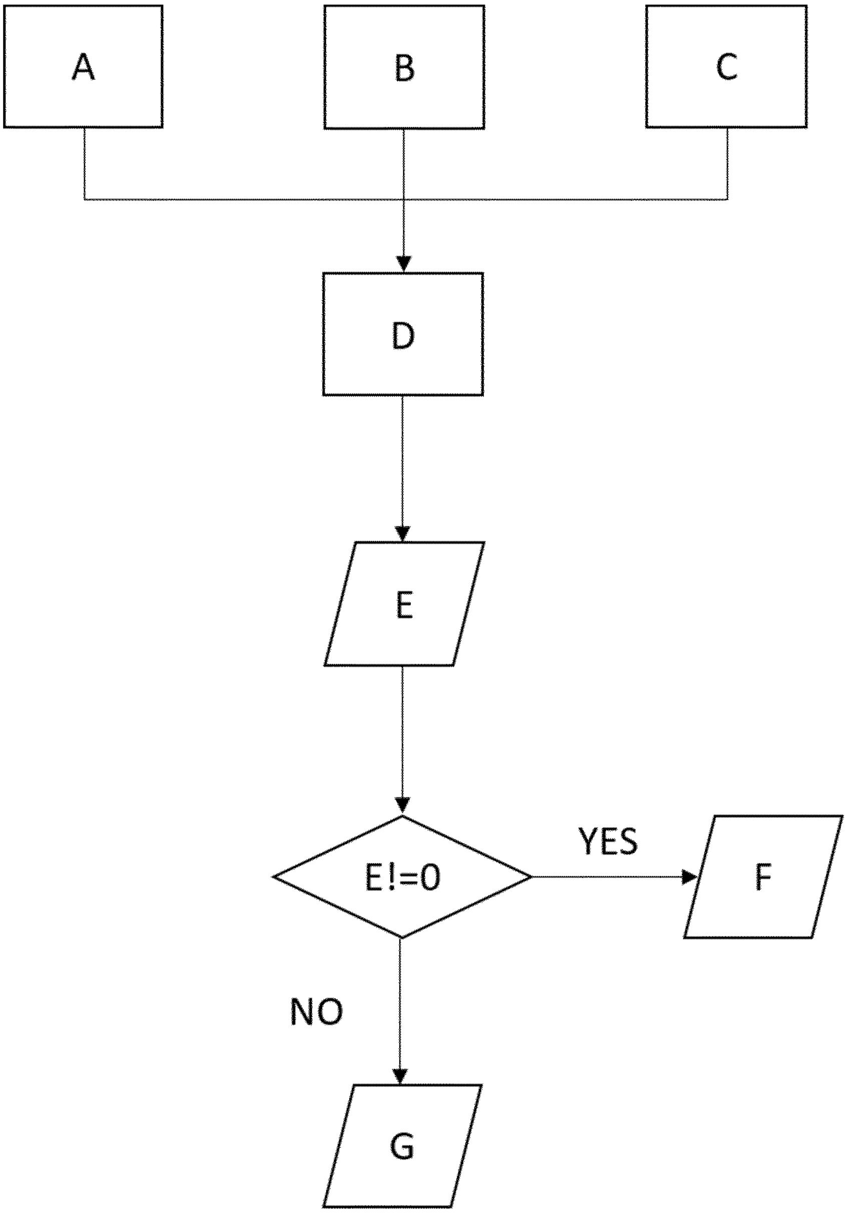


Fig. 2

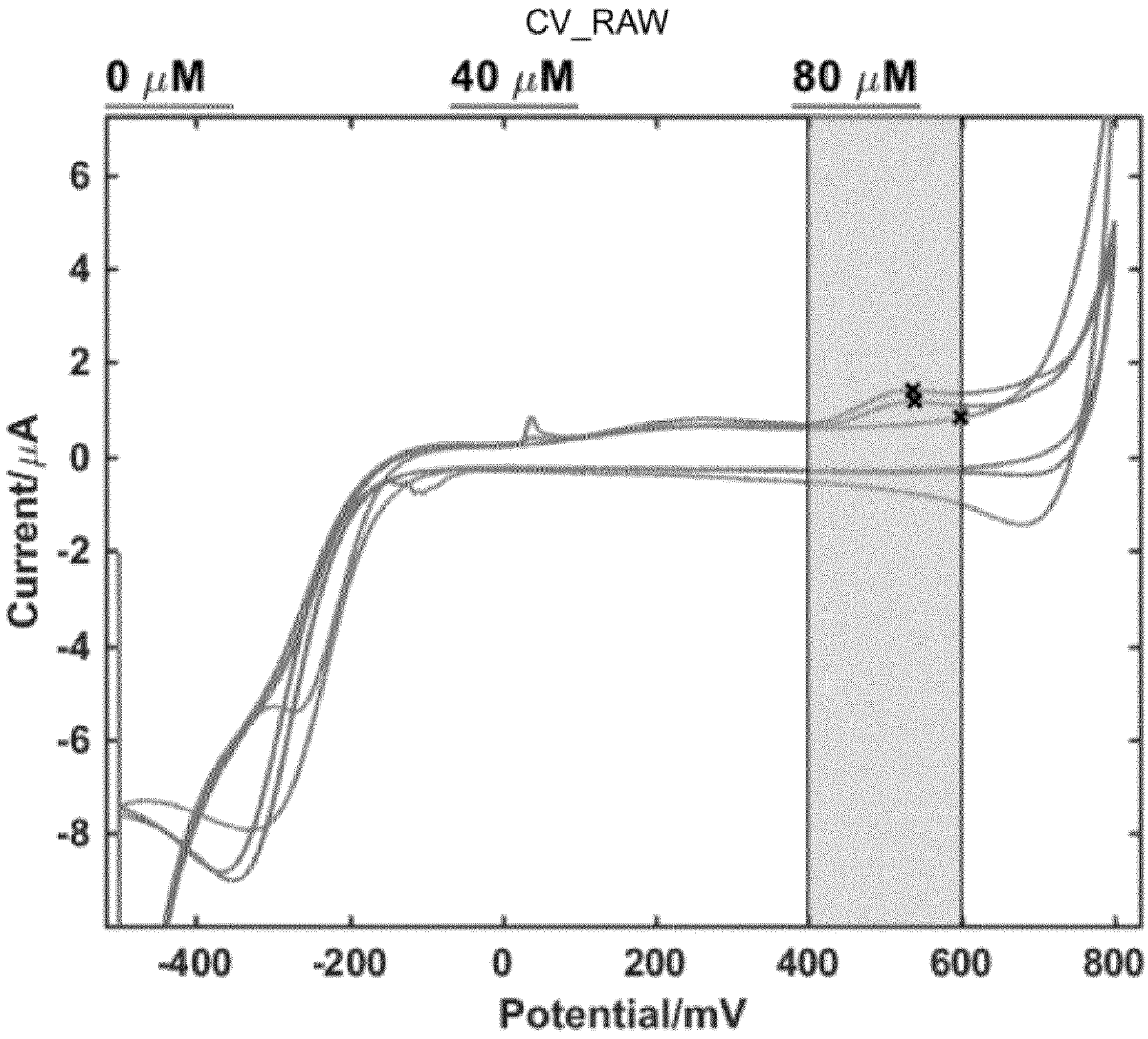
CV_RAW
0 μM
40 μM
80 μM
6
4
2
0
-2
-4
-6
-8
Current/μA
-400
-200
0
200
400
600
800
Potential/mV

Fig. 3

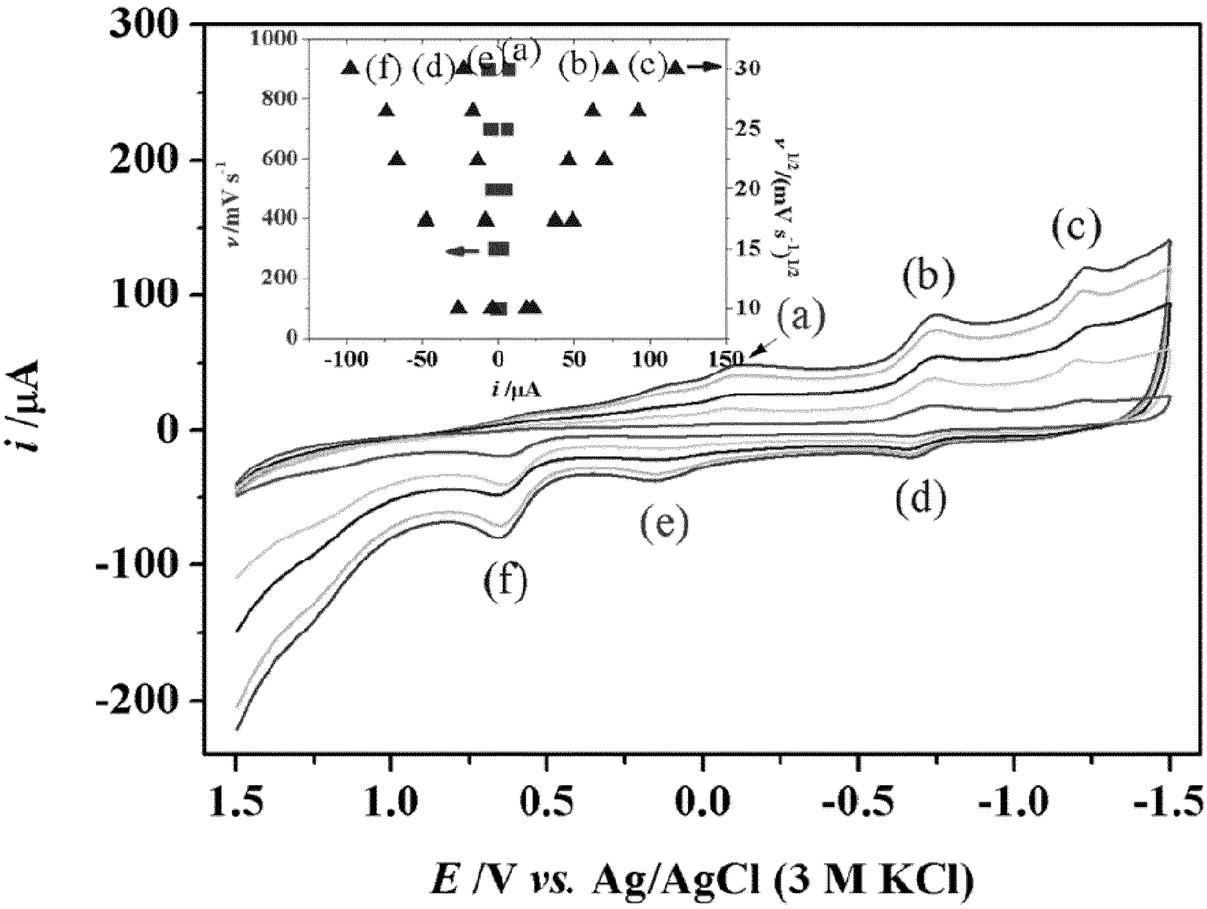
300
200
100
0
-100
-200
i /μA
1.5
1.0
0.5
0.0
-0.5
-1.0
-1.5
E /V vs. Ag/AgCl (3 M KCl)
(a)
(b)
(c)
(d)
(e)
(f)
1000
800
600
400
200
0
-100
-50
0
50
100
150
i /μA
v /mV s-1
v1/2/(mV s-1)1/2
30
25
20
15
10

Fig. 4

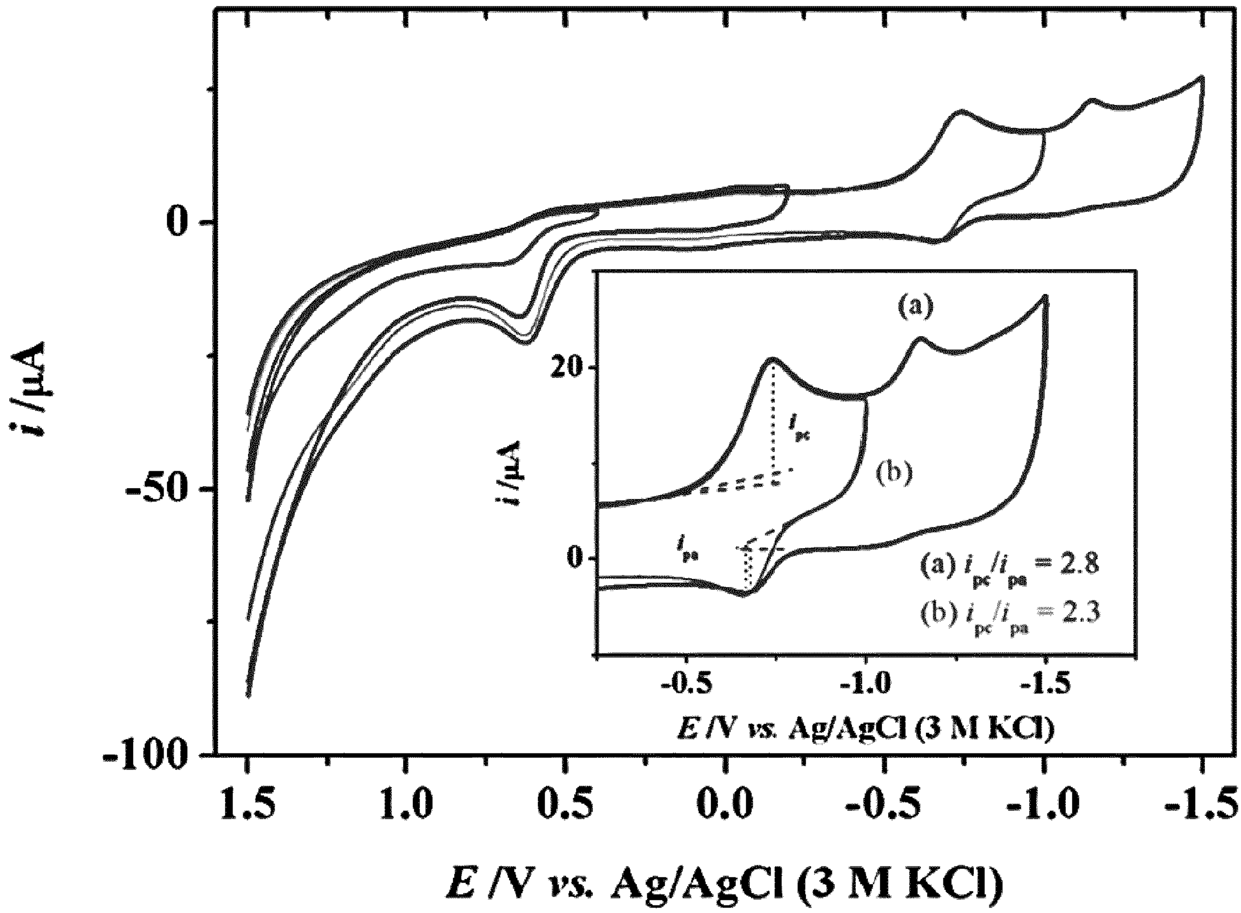
0
-50
-100
i /μA
1.5
1.0
0.5
0.0
-0.5
-1.0
-1.5
E /V vs. Ag/AgCl (3 M KCl)
(a)
20
0
i /μA
$i_{pc}$
$i_{pa}$
(b)
(a) $i_{pc}/i_{pa}$ = 2.8
(b) $i_{pc}/i_{pa}$ = 2.3
-0.5
-1.0
-1.5
E /V vs. Ag/AgCl (3 M KCl)

Fig. 5

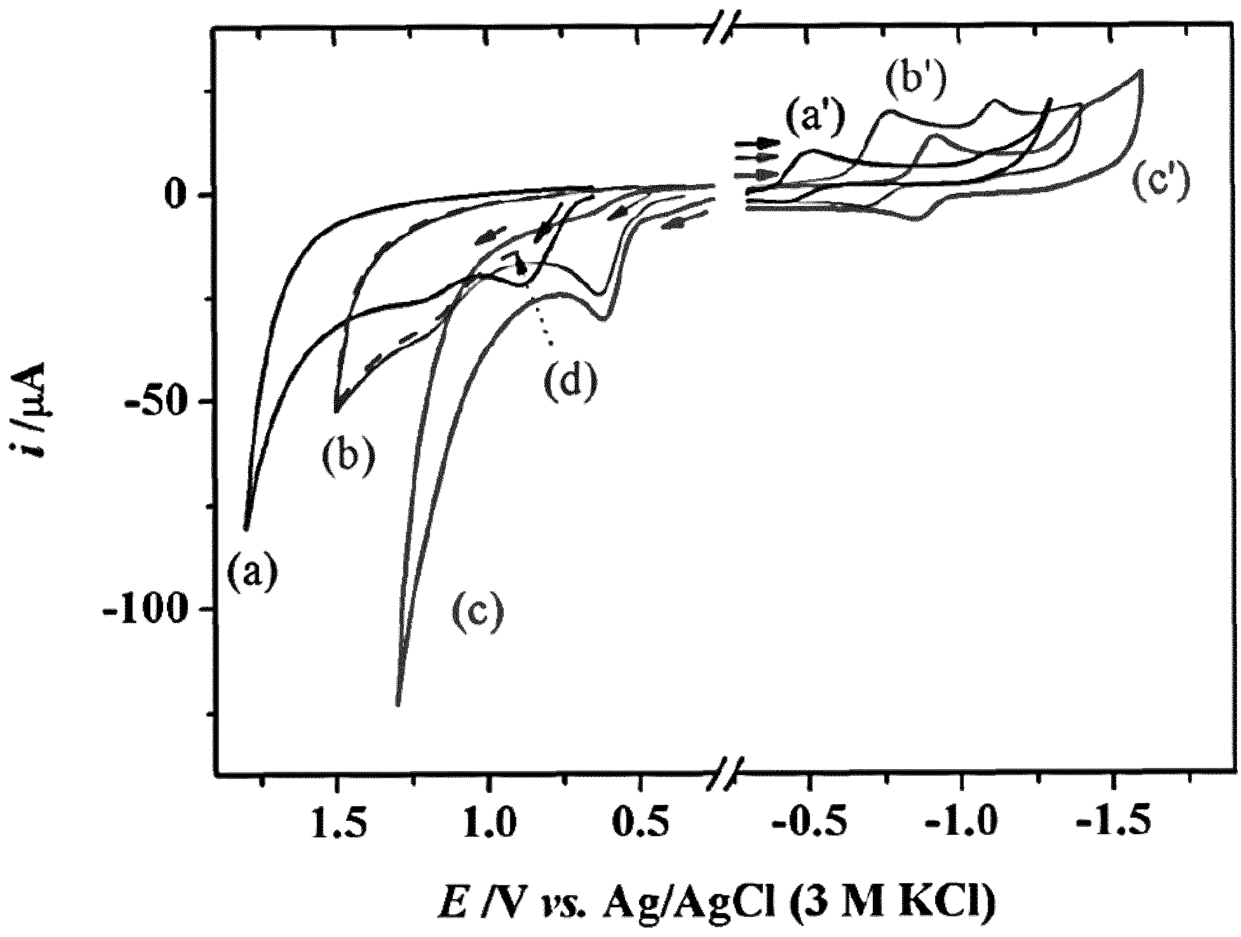


Fig. 6

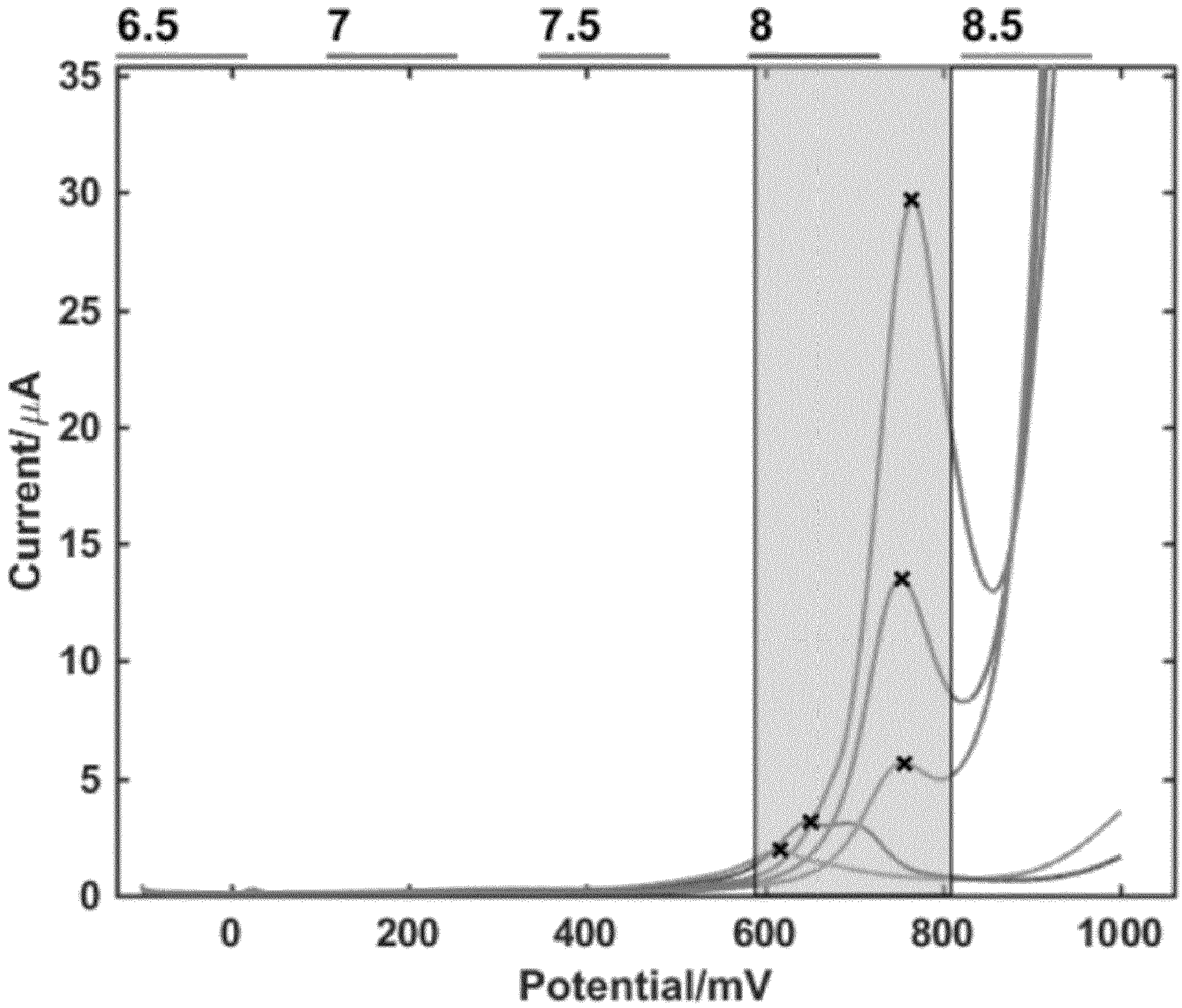
6.5
7
7.5
8
8.5
35
30
25
20
15
10
5
0
Current/μA
0
200
400
600
800
1000
Potential/mV

Fig. 7a

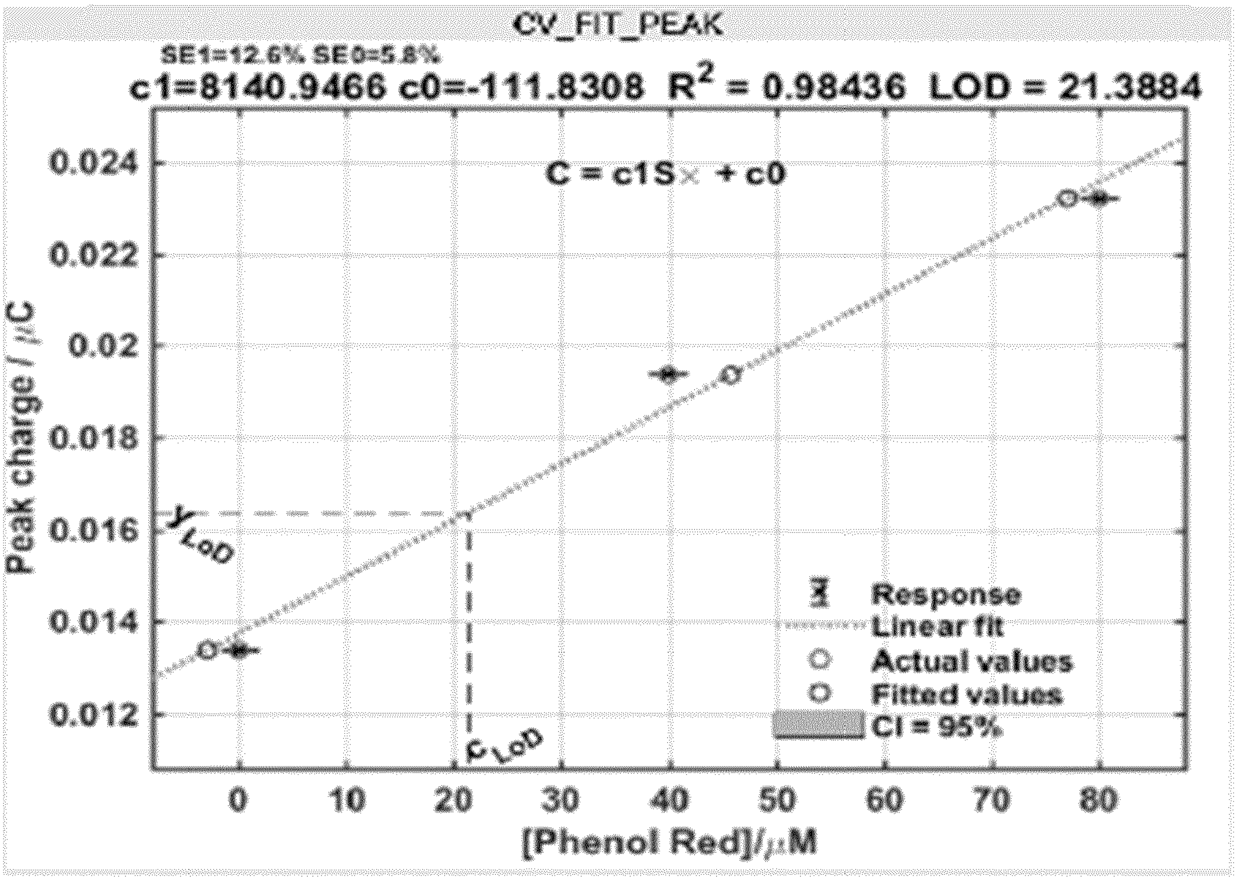
CV_FIT_PEAK
SE1=12.6% SE0=5.8%
c1=8140.9466 c0=-111.8308 R2 = 0.98436 LOD = 21.3884
C = c1S× + c0
Peak charge / μC
0.024
0.022
0.02
0.018
0.016
0.014
0.012
yLoD
cLoD
Response
Linear fit
Actual values
Fitted values
CI = 95%
0
10
20
30
40
50
60
70
80
[Phenol Red]/μM

Fig 7b

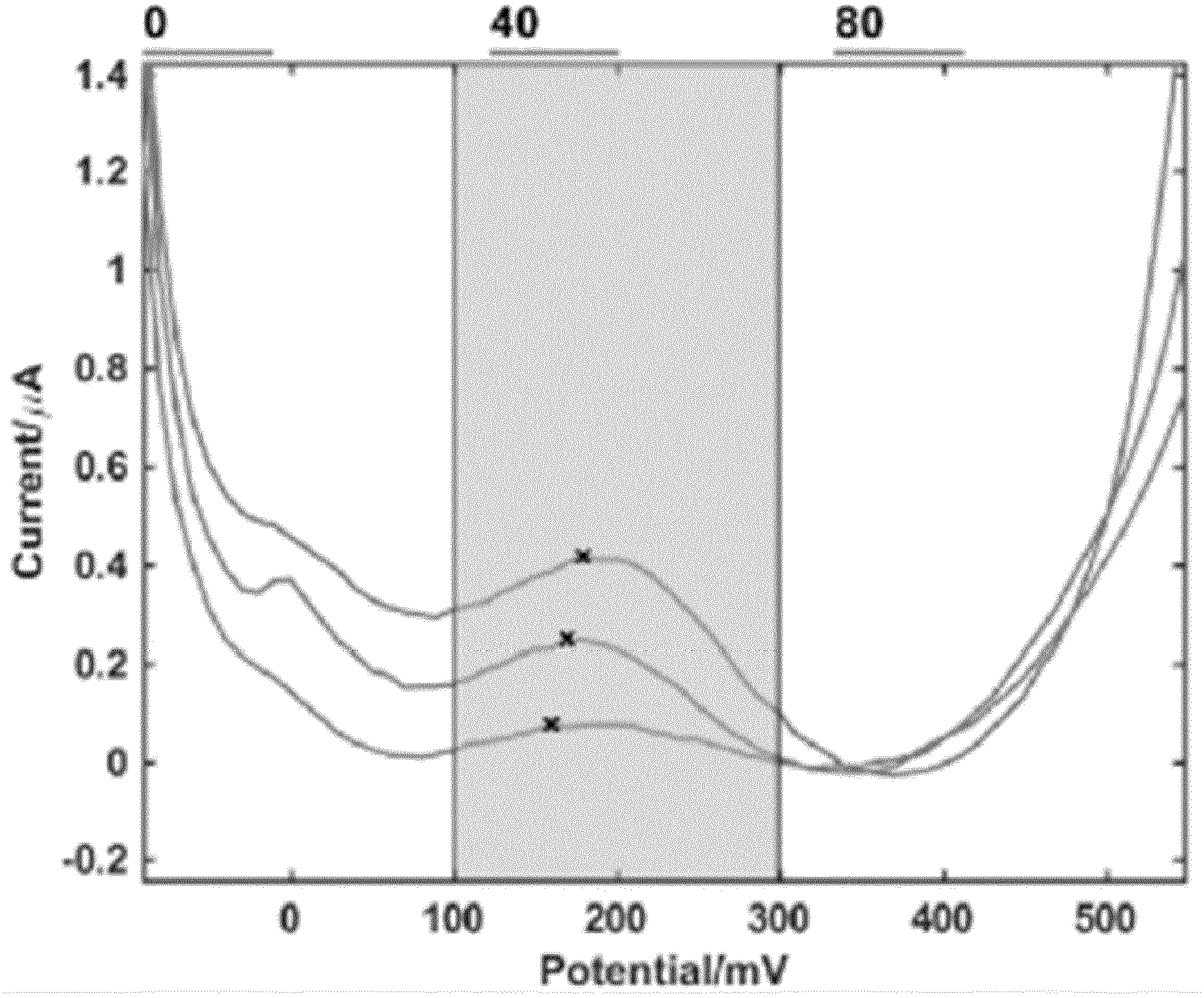
0
40
80
1.4
1.2
1
0.8
0.6
0.4
0.2
0
-0.2
Current/μA
0
100
200
300
400
500
Potential/mV

Fig. 7c

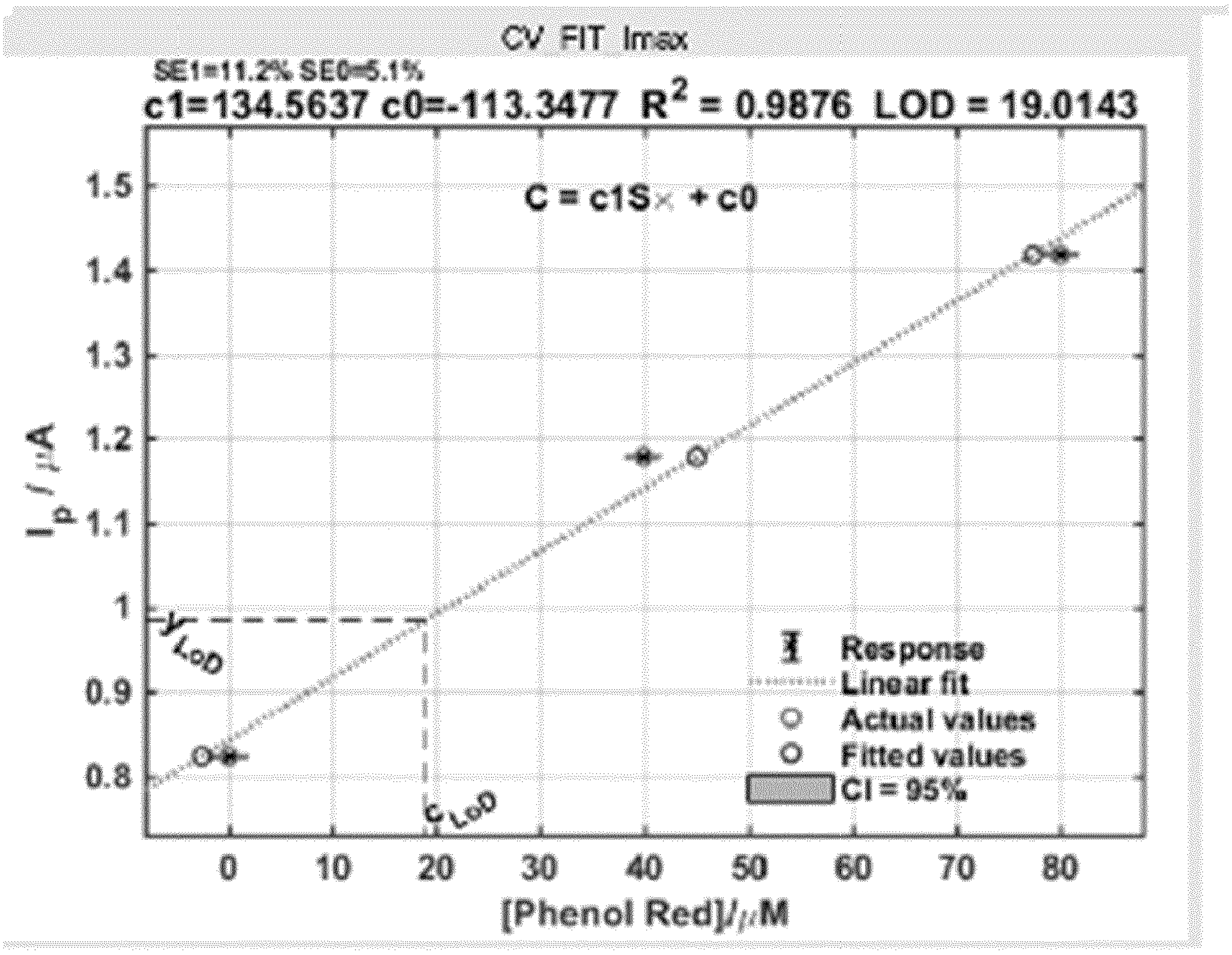
CV FIT Imax
SE1=11.2% SE0=5.1%
c1=134.5637 c0=-113.3477 R2 = 0.9876 LOD = 19.0143
C = c1S× + c0
Ip / μA
1.5
1.4
1.3
1.2
1.1
1
0.9
0.8
yLoD
cLoD
Response
Linear fit
Actual values
Fitted values
CI = 95%
0
10
20
30
40
50
60
70
80
[Phenol Red]/μM

Fig. 7d

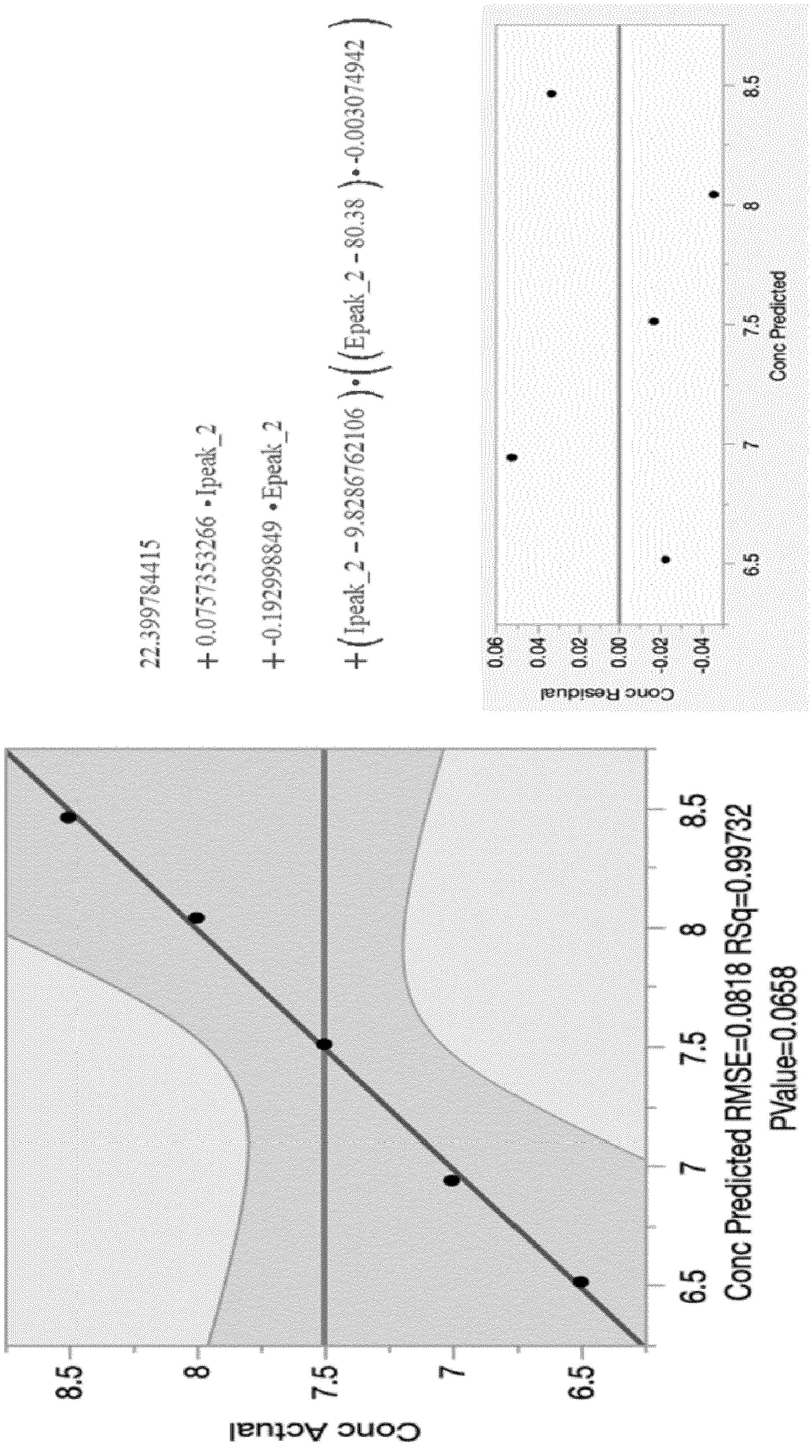
22.399784415
+ 0.0757353266 • Ipeak_2
+ -0.192998849 • Epeak_2
+ (Ipeak_2 − 9.8286762106) • ((Epeak_2 − 80.38) • -0.003074942)
Conc Actual
Conc Predicted RMSE=0.0818 RSq=0.99732
PValue=0.0658
Conc Residual
Conc Predicted

Fig. 8

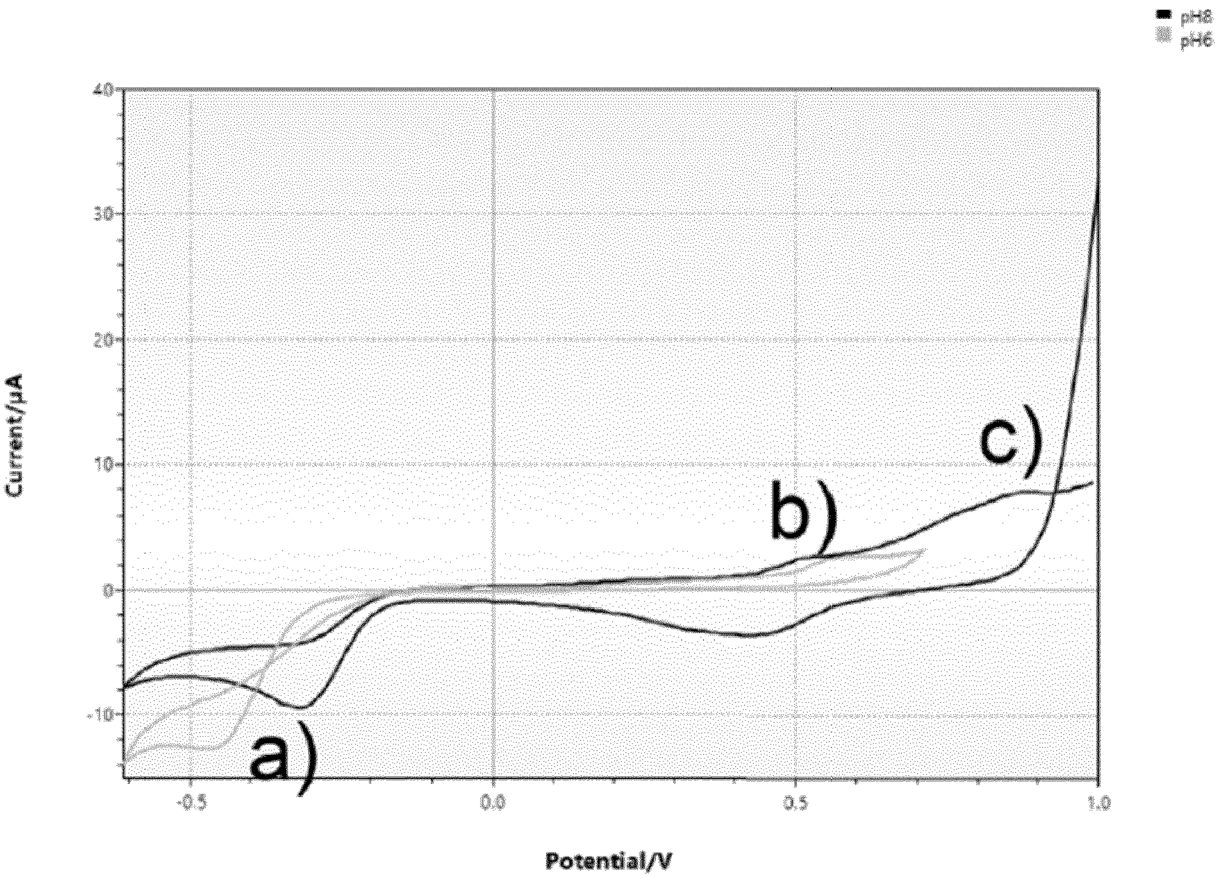


Fig. 9

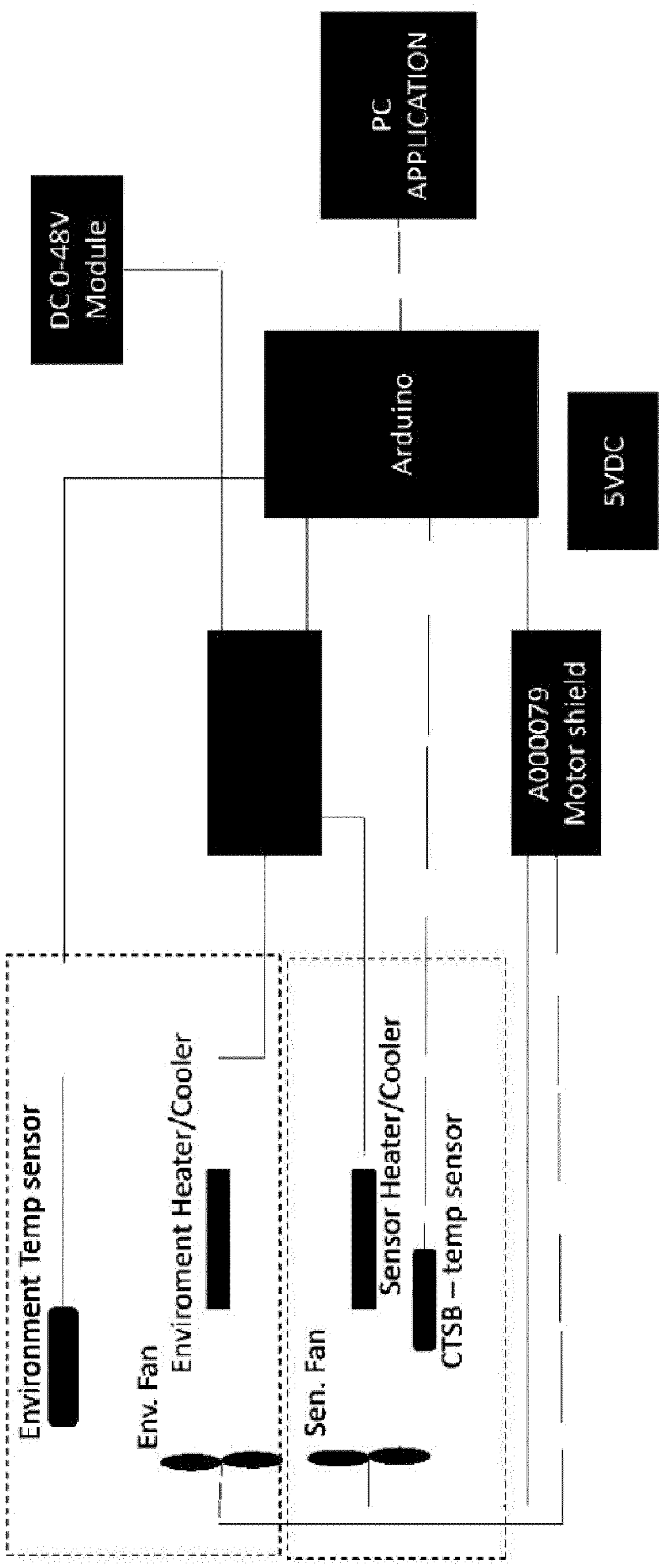
PC APPLICATION
DC 0-48V Module
Arduino
5VDC
A000079 Motor shield
Environment Temp sensor
Enviroment Heater/Cooler
Env. Fan
Sensor Heater/Cooler
CTSB – temp sensor
Sen. Fan

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017209920 A
- WO 2014031783 A
- GB 2501769 A
- EP 20197572
- EP 20168019


### Non-patent literature cited in the description


- **THOMPSON et al.** *Nucleic Acids Res,* 1994, vol. 22, 4673-80
- **NARANG et al.** *Methods Enzymol.,* 1979, vol. 68, 90
- **BROWN et al.** *Methods Enzymol.,* 1979, vol. 68, 109